# EUROPÄISCHE PATENTSCHRIFT

(11) **EP 3 645 527 B1**
(45) Veröffentlichungstag und Bekanntmachung des Hinweises auf die Patenterteilung: **04.11.2026**
(21) Anmeldenummer: 18732109.6
(22) Anmeldetag: 25.06.2018
(51) Int. Cl.: C07D 403/04, C07D 407/04, C07D 251/24

(54) **VERFAHREN ZUR HERSTELLUNG SUBSTITUIERTER STICKSTOFFHALTIGER HETEROCYCLEN**
METHOD FOR PRODUCING SUBSTITUTED NITROGEN-CONTAINING HETEROCYCLES
PROCÉDÉS DE PRÉPARATION D'HÉTÉROCYCLES SUBSTITUÉS CONTENANT DE L'AZOTE

(30) Priorität: 26.06.2017 EP 17177923
(43) Veröffentlichungstag der Anmeldung: 06.05.2020
(73) Patentinhaber: Merck Patent GmbH, 64293 Darmstadt (DE)
(72) Erfinder: KAUFHOLD, Oliver, 64297 Darmstadt (DE); JASPER, Christian, 63500 Seligenstadt (DE); HAHN, Helmut, 65428 Ruesselsheim (DE)
(74) Vertreter: Merck Patent Association
(86) Internationale Anmeldenummer: PCT/EP2018/066910
(87) Internationale Veröffentlichungsnummer: WO 2019/002183

(56) Entgegenhaltungen:
- EP-A1- 1 604 988
- EP-A2- 0 941 989
- WO-A1-2010/072300
- US-A1- 2010 093 731
- US-A1- 2015 266 834
- US-A1- 2017 057 943
- GARG NEIL K ET AL: "The first total synthesis of dragmacidin d", JOURNAL OF THE AMERICAN CHEMICAL SOCIETY, AMERICAN CHEMICAL SOCIETY, vol. 124, no. 44, 6 November 2002 (2002-11-06), pages 13179 - 13184, XP009143391, ISSN: 0002-7863, DOI: 10.1021/JA027822B
- OLESYA M. KUZMINA ET AL: "Ligand-Accelerated Iron- and Cobalt-Catalyzed Cross-Coupling Reactions between N-Heteroaryl Halides and Aryl Magnesium Reagents", ANGEWANDTE CHEMIE INTERNATIONAL EDITION, vol. 52, no. 18, 26 April 2013 (2013-04-26), pages 4945 - 4949, XP055338962, ISSN: 1433-7851, DOI: 10.1002/anie.201210235
- XIAO-JIAN LI ET AL: "Nickel-Catalyzed Suzuki-Miyaura Coupling of Heteroaryl Ethers with Arylboronic Acids", JOURNAL OF ORGANIC CHEMISTRY, vol. 78, no. 10, 17 May 2013 (2013-05-17), US, pages 5078 - 5084, XP055492208, ISSN: 0022-3263, DOI: 10.1021/jo4005537
- LI XU ET AL: "Synthesis and spectroscopic properties of propeller type 2,4,6-tri(anthracen-9-yl)-1,3,5-triazine", RSC ADVANCES, vol. 5, no. 64, 1 January 2015 (2015-01-01), pages 51745 - 51749, XP055492387, ISSN: 2046-2069, DOI: 10.1039/C5RA08833J
- LI XU ET AL: "Electronic Supplementary Information (ESI) for: Synthesis and Spectroscopic Properties of Propeller Typed 2,4,6- Tri(anthracen-9-yl)-1,3,5-triazine", 1 January 2015 (2015-01-01), XP055492625, Retrieved from the Internet <URL:http://www.rsc.org/suppdata/c5/ra/c5ra08833j/c5ra08833j1.pdf> [retrieved on 20180716]

## Beschreibung

Die vorliegende Erfindung beschreibt Verfahren zur Herstellung von substituierten stickstoffhaltigen Heterocyclen, insbesondere zur Verwendung in elektronischen Vorrichtungen.

Elektronische Vorrichtungen, welche organische, metallorganische und/oder polymere Halbleiter enthalten, gewinnen zunehmend an Bedeutung, wobei diese aus Kostengründen und aufgrund ihrer Leistungsfähigkeit in vielen kommerziellen Produkten eingesetzt werden. Als Beispiele seien hier Ladungstransportmaterialien auf organischer Basis (z.B. Lochtransporter auf Triarylamin-Basis) in Kopiergeräten, organischen oder polymeren Leuchtdioden (OLEDs oder PLEDs) und in Anzeige- und Displayvorrichtungen oder organische Photorezeptoren in Kopierern genannt. Organische Solarzellen (O-SC), organische FeldeffektTransistoren (O-FET), organische Dünnfilm-Transistoren (O-TFT), organische Schaltelemente (O-IC), organische optische Verstärker und organische Laserdioden (O-Laser) sind in einem fortgeschrittenen Entwicklungsstand und können in der Zukunft große Bedeutung erlangen.

Zur Herstellung dieser Vorrichtungen werden vielfach organisch funktionelle Materialien eingesetzt, die substituierte stickstoffhaltige Heterocyclen enthalten. Ein Überblick über diese Reaktionen findet sich in *Houben-Weyl - Methods of Organic Chemistry, Vol. E9c.*

Unsymmetrische Triazine werden bevorzugt durch Umsetzung von Trichlor-Triazin (Cyanurchlorid) erhalten. Hierzu werden die Chloratome sukzessive über ein oder zwei Zwischenstufen durch aromatische Substituenten ausgetauscht. Dies kann durch klassische Grignard-Reagenzien (zB. EP 577 559 A, Org. Lett., Vol. 10, No. 5, 2008) oder durch Suzuki-Kupplungen (EP 2 423 209 A) durchgeführt werden.

Diese Verfahren liefern nur in Ausnahmefällen akzeptable Selektivitäten und Ausbeuten. Im Allgemeinen werden lediglich geringe Ausbeuten und unzureichende Selektivitäten erreicht. So werden beispielsweise in EP 577 559 A Verfahren beschrieben, bei denen die Produkte im Wesentlichen mit nur bis zu einer 93%igen Reinheit, bei einer Ausbeute im Bereich 60-92%, erhalten werden. Eine weitere Aufreinigung würde zu einer spürbar geringeren Ausbeute führen.

Des weiteren können Triazinderivate über einen oxidativen Aufbau des Triazinringes (Tet. Lett. 2014, 55, 6976) erhalten werden. Bei dieser Methode wird ein aromatischer Ring geopfert. Bei der Einführung komplexer aromatischer Ringe wird dieses Verfahren schnell ökonomisch unrentabel.

Darüber hinaus sind Methoden bekannt, bei denen Triazinderivate über Imine mit Amidinen (Journal of Materials Chemistry, 2007 , vol. 17, 3714 - 3719; EP2415769) dargestellt werden. Allerdings sind nur "simple" Beispiele bekannt. Diese Route benötigt "komplexe" Amidine und/oder "komplexe" aromatische Aldehyde, die im Allgemeinen nicht kommerziell erhältlich sind, so dass diese Methode bisher nicht generell angewendet werden kann.

Triazinderivate können weiterhin über Lewis-Säure katalysierte Cyclisierung von 2eq. Nitril mit 1 eq. Säurechlorid und anschließender Ammonolyse (Chem. Ber. 1965, 334) hergestellt werden. Nachteil dieser Variante ist die einerseits die schwache Selektivität, es enststeht vielfach ein Produktgemisch, sowie die schlechte Verfügbarkeit komplexer Nitrile und Säurechloride.

US 2015/0266834 A1 offenbart ein Verfahren zur Herstellung von unsymmetrischen Triazinen, bei welchem die Titelverbindungen durch eine Sequenz aus zwei Suzuki-Kupplungen mit anschließender Hydrolyse einer Alkoxygruppe OR⁶ erhalten werden:

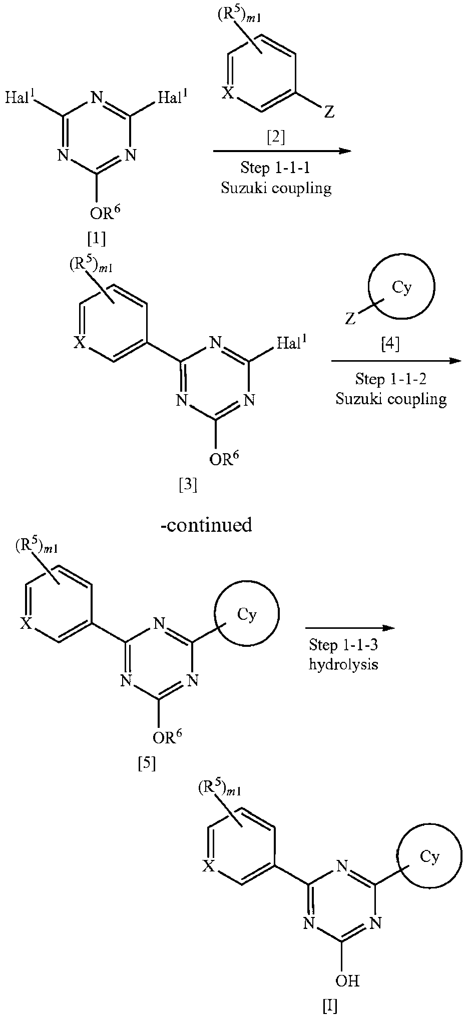

WO 2017/016630 A1 offenbart ein Verfahren zur Herstellung von substituierten Triazinen, bei dem unter anderen Grignard-Reagenzien verwendet werden können:

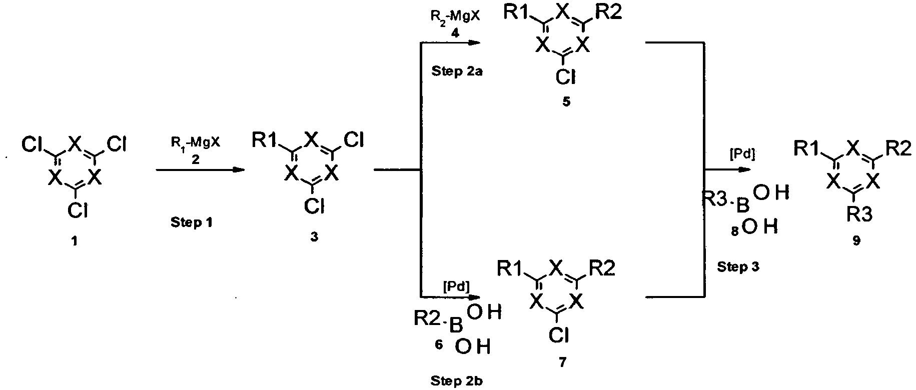

Es besteht ein Bedürfnis nach einem verbesserten Verfahren zur Herstellung von substituierten stickstoffhaltigen Heterocyclen, die insbesondere zur Verwendung in elektronischen Vorrichtungen geeignet sind. Eine weitere Aufgabe bestand insbesondere darin, ein Verfahren bereitzustellen, welches zu einer hohen Selektivität bei einer hohen Ausbeute führt. Ferner sollte das Verfahren mit relativ geringen Kosten verbunden sein. Weiterhin sollte die Notwendigkeit vermindert werden, aufwändige Reinigungsverfahren durchzuführen.

Überraschend wurde gefunden, dass bestimmte, nachfolgend näher beschriebene Verfahren diese Aufgaben lösen und den Nachteil aus dem Stand der Technik beseitigen. Durch die dargelegten Verfahren können insbesondere Verbesserungen hinsichtlich der Selektivität, der Ausbeute und der Kosten zur Herstellung von substituierten stickstoffhaltigen Heterocyclen erzielt werden, die insbesondere zur Verwendung in elektronischen Vorrichtungen geeignet sind.

Gegenstand der vorliegenden Erfindung ist daher ein Verfahren zur Herstellung von unsymmetrisch substituierten stickstoffhaltigen Heterocyclen umfassend die Schritte:
A) Umsetzung eines Edukts gemäß der folgenden Formel (I) 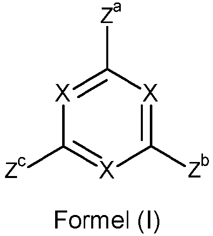
   wobei für die verwendeten Symbole und Indizes gilt:
      - Z^{a}, Z^{b}, Z^{c}: ist Halogen oder OR;
      - X: ist bei jedem Auftreten gleich oder verschieden CR¹ oder N, vorzugsweise N, mit der Maßgabe, dass mindestens zwei der Symbole X für N und speziell bevorzugt alle Symbole X für N stehen;
      - R,: ist bei jedem Auftreten gleich oder verschieden, S(=O)₂R², eine geradkettige Alkylgruppe mit 1 bis 20 C-Atomen, eine verzweigte oder cyclische Alkylgruppe mit 3 bis 20 C-Atomen, wobei die lineare, verzweigte oder cyclische Alkylgruppe jeweils mit einem oder mehreren Resten R² substituiert sein kann, oder ein aromatisches oder heteroaromatisches Ringsystem mit 5 bis 40 aromatischen Ringatomen, das jeweils durch einen oder mehrere Reste R² substituiert sein kann;
      - R¹: ist bei jedem Auftreten gleich oder verschieden eine geradkettige Alkylgruppe mit 1 bis 20 C-Atomen, eine verzweigte oder cyclische Alkylgruppe mit 3 bis 20 C-Atomen, wobei die lineare, verzweigte oder cyclische Alkylgruppe jeweils mit einem oder mehreren Resten R² substituiert sein kann, oder ein aromatisches oder heteroaromatisches Ringsystem mit 5 bis 40 aromatischen Ringatomen, das jeweils durch einen oder mehrere Reste R² substituiert sein kann, dabei können zwei oder mehrere Reste R¹ miteinander ein Ringsystem bilden;
      - R²: ist bei jedem Auftreten gleich oder verschieden H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R³ substituiert sein kann, wobei eine oder mehrere nicht benachbarte CH₂-Gruppen durch R³C=CR³, C≡C, Si(R³)₂, C=O, NR³, O, S oder CONR³ ersetzt sein können, oder ein aromatisches oder heteroaromatisches Ringsystem mit 5 bis 40 aromatischen Ringatomen, das jeweils durch einen oder mehrere Reste R³ substituiert sein kann, oder eine Aryloxy- oder Heteroaryloxygruppe mit 5 bis 40 aromatischen Ringatomen, die durch einen oder mehrere Reste R³ substituiert sein kann, oder eine Aralkyl- oder Heteroaralkylgruppe mit 5 bis 40 aromatischen Ringatomen, die durch einen oder mehrere Reste R³ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R³ substituiert sein kann; dabei können zwei oder mehrere Reste R² miteinander ein Ringsystem bilden;
      - R³: ist bei jedem Auftreten gleich oder verschieden H, D, F oder ein aliphatischer, aromatischer und/oder heteroaromatischer organischer Rest, insbesondere ein Kohlenwasserstoffrest, mit 1 bis 20 C-Atomen, in dem auch ein oder mehrere H-Atome durch F ersetzt sein können, dabei können zwei oder mehrere Substituenten R³ auch miteinander ein mono- oder polycyclisches Ringsystem bilden;
   mit der Maßgabe, dass die Gruppe Z^{a} ungleich der Gruppe Z^{b} oder Z^{c} und mindestens eine der Gruppen Z^{a}, Z^{b}, Z^{c} ein Halogen ist;
   mit einer ersten reaktiven aromatischen oder heteroaromatischen Verbindung (A) unter Erhalt eines Zwischenprodukts (ZA), wobei die erste reaktive aromatische oder heteroaromatische Verbindung (A) eine Aryl- oder Heteroarylverbindung der Formel (A) ist 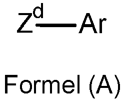
   wobei für die verwendeten Symbole und Indizes gilt:
      - Ar: ist bei jedem Auftreten gleich oder verschieden eine Aryl- oder Heteroarylgruppe mit 5 bis 60 aromatischen Ringatomen, die mit einem oder mehreren Resten R¹ substituiert sein kann, wobei zwei Reste R auch miteinander ein Ringsystem bilden können;
      - Z^{d}: ist eine reaktive Gruppe B(R^{1a})₂;
      - R^{1a}: ist bei jedem Auftreten gleich oder verschieden OH, eine geradkettige Alkyl- oder Alkoxygruppe mit 1 bis 20 C-Atomen, eine verzweigte oder cyclische Alkyl- oder Alkoxygruppe mit 3 bis 20 C-Atomen, wobei die lineare, verzweigte oder cyclische Alkyl- oder Alkoxygruppe jeweils mit einem oder mehreren Resten R² substituiert sein kann, oder ein aromatisches oder heteroaromatisches Ringsystem mit 5 bis 40 aromatischen Ringatomen, das jeweils durch einen oder mehrere Reste R² substituiert sein kann, dabei können zwei oder mehrere Reste R^{1a} miteinander ein Ringsystem bilden
      wobei R² die für die Formel (I) genannte Bedeutung aufweist;
B) Umsetzung des Zwischenprodukts (ZA) mit einer zweiten reaktiven aromatischen oder heteroaromatischen Verbindung (B), die sich von der ersten reaktiven aromatischen oder heteroaromatischen Verbindung (A) unterscheidet, unter Erhalt einer unsymmetrisch substituierten stickstoffhaltigen heterocyclischen Verbindung,

dadurch gekennzeichnet, dass die in Schritt B) umgesetzte Gruppe eine Gruppe der Formel OR ist und die in Schritt B) eingesetzte zweite reaktive aromatischen oder heteroaromatischen Verbindung (B) eine metallorganische Aryl- oder Heteroarylverbindung gemäß der Formel (B) ist

   M-Ar Formel (B)
wobei für die verwendeten Symbole gilt:
   - Ar: ist bei jedem Auftreten gleich oder verschieden eine Aryl- oder Heteroarylgruppe mit 5 bis 60 aromatischen Ringatomen, die mit einem oder mehreren Resten R¹ substituiert sein kann, wobei zwei Reste R¹ auch miteinander ein Ringsystem bilden können und R¹ die für die Formel (I) genannte Bedeutung aufweist;
   - M: ist ein Metallatom, vorzugsweise Li, oder ein metallorganischer Rest, vorzugsweise MgBr oder MgCl,
und wobei die Umsetzung mit der metallorganischen Aryl- oder Heteroarylverbindung ohne den Zusatz von Übergangsmetall-Katalysatoren durchgeführt wird.

In einer bevorzugten Ausführungsform sind R und R¹ bei jedem Auftreten gleich oder verschieden eine geradkettige Alkylgruppe mit 1 bis 20 C-Atomen, eine verzweigte oder cyclische Alkylgruppe mit 3 bis 20 C-Atomen, wobei die lineare, verzweigte oder cyclische Alkylgruppe jeweils mit einem oder mehreren Resten R² substituiert sein kann oder ein aromatisches oder heteroaromatisches Ringsystem mit 5 bis 40 aromatischen Ringatomen, das jeweils durch einen oder mehrere Reste R² substituiert sein kann, dabei können zwei oder mehrere Reste R¹ miteinander ein Ringsystem bilden.

In einer ganz bevorzugten Ausführungsform sind R und R¹ bei jedem Auftreten gleich oder verschieden eine geradkettige Alkylgruppe mit 1 bis 20 C-Atomen, eine verzweigte oder cyclische Alkylgruppe mit 3 bis 20 C-Atomen, wobei die lineare, verzweigte oder cyclische Alkylgruppe jeweils mit einem oder mehreren Resten R² substituiert sein kann oder ein aromatisches Ringsystem mit 6 bis 40 aromatischen Ringatomen, das jeweils durch einen oder mehrere Reste R² substituiert sein kann, dabei können zwei oder mehrere Reste R¹ miteinander ein Ringsystem bilden.

In einer besonders bevorzugten Ausführungsform sind R und R¹ bei jedem Auftreten gleich oder verschieden eine geradkettige Alkylgruppe mit 1 bis 20 C-Atomen, eine verzweigte oder cyclische Alkylgruppe mit 3 bis 20 C-Atomen, wobei die lineare, verzweigte oder cyclische Alkylgruppe jeweils mit einem oder mehreren Resten R² substituiert sein kann oder ein aromatisches Ringsystem mit 6 bis 40 aromatischen Ringatomen, das jeweils durch einen oder mehrere Reste R² substituiert sein kann.

Benachbarte Kohlenstoffatome im Sinne der vorliegenden Erfindung sind Kohlenstoffatome, die direkt miteinander verknüpft sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten".

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

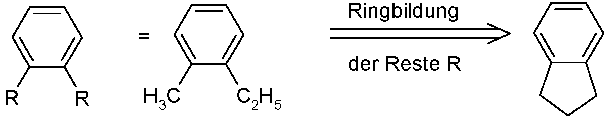

Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

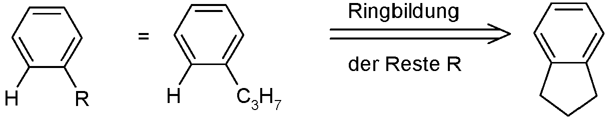

Eine kondensierte Arylgruppe, ein kondensiertes aromatisches Ringsystem oder ein kondensiertes heteroaromatisches Ringsystem im Sinne der vorliegenden Erfindung ist eine Gruppe, in der zwei oder mehr aromatische Gruppen über eine gemeinsame Kante aneinander ankondensiert, d. h. anelliert, sind, so dass beispielsweise zwei C-Atome zu den mindenstens zwei aromatischen oder heteroaromatischen Ringen zugehören, wie beispielsweise im Naphthalin. Dagegen ist beispielsweise Fluoren keine kondensierte Arylgruppe im Sinne der vorliegenden Erfindung, da im Fluoren die beiden aromatischen Gruppen keine gemeinsame Kante aufweisen. Entsprechende Definitionen gelten für Heteroarylgruppen sowie für kondensierte Ringsysteme, die auch Heteroatome enthalten können, jedoch nicht müssen.

Eine Arylgruppe im Sinne dieser Erfindung enthält 6 bis 60 C-Atome, vorzugsweise 6 bis 40 C-Atome; eine Heteroarylgruppe im Sinne dieser Erfindung enthält 2 bis 60 C-Atome, vorzugsweise 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl- oder Heteroarylgruppe, beispielsweise Naphthalin, Anthracen, Phenanthren, Chinolin, Isochinolin, etc., verstanden.

Ein aromatisches Ringsystem im Sinne dieser Erfindung enthält 6 bis 60 C-Atome, vorzugsweise 6 bis 40 C-Atome im Ringsystem. Ein heteroaromatisches Ringsystem im Sinne dieser Erfindung enthält 1 bis 60 C-Atome, vorzugsweise 1 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie 9,9'-Spirobifluoren, 9,9-Diarylfluoren, Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl- oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer C₁- bis C₂₀-Alkylgruppe, in der auch einzelne H-Atome oder CH₂-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer C₁- bis C₄₀-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 60 aromatischen Ringatomen, vorzugsweise 5 - 40 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Isochinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Ferner kann vorgesehen sein, dass in der Formel (I) zwei der Gruppen Z^{a}, Z^{b}, Z^{c} gleich sind. Bevorzugt kann mindestens eine der Gruppen Z^{a}, Z^{b}, Z^{c} eine Alkoxy-Gruppe oder eine Aryloxy-Gruppe mit bevorzugt 1 bis 20 C-Atomen, besonders bevorzugt 1 bis 10 C-Atomen, insbesondere eine Methoxy-, Ethoxy-, Propoxy- oder Phenoxy-Gruppe, vorzugsweise eine Methoxy-, Ethoxy-, Phenoxy-Gruppe, besonders bevorzugt eine Methoxy- oder Phenoxy-Gruppe sein. Speziell bevorzugt kann mindestens eine der Gruppen Z^{a}, Z^{b}, Z^{c} Cl, Br oder I, vorzugsweise Cl oder Br, besonders bevorzugt Cl sein.

In einer bevorzugten Ausführungsform kann vorgesehen sein, dass mindestens eine der Gruppen Z^{a}, Z^{b}, Z^{c} Cl ist und mindestens eine der Gruppen Z^{a}, Z^{b}, Z^{c} eine Methoxy- oder Phenoxy-Gruppe ist, vorzugsweise zwei der Gruppen Z^{a}, Z^{b}, Z^{c} eine Methoxy- oder Phenoxy-Gruppe darstellen.

Bevorzugt kann ein Edukt gemäß der folgenden Formel (IV) eingesetzt werden 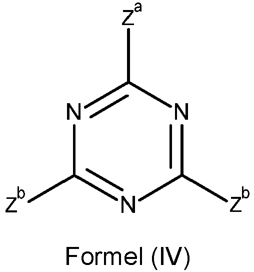 wobei die Symbole die zuvor, insbesondere für Formel (I) genannte Bedeutung aufweisen und die zwei Reste Z^{b} gleich sind.

Weiterhin sind Verbindungen gemäß Formel (IV) bevorzugt, bei denen Z^{a} OR darstellt, wobei R die zuvor, insbesondere für Formel (I) genannte Bedeutung aufweist, wobei Z^{a} bevorzugt eine Alkoxy-Gruppe oder eine Aryloxy-Gruppe mit bevorzugt 1 bis 20 C-Atomen, besonders bevorzugt 1 bis 10 C-Atomen, insbesondere eine Methoxy-, Ethoxy-, Propoxy- oder Phenoxy-Gruppe, vorzugsweise eine Methoxy-, Ethoxy- oder Phenoxy-Gruppe, besonders bevorzugt eine Methoxy- oder Phenoxy-Gruppe ist, und Z^{b} ein Halogen ist, vorzugsweise Cl, Br oder I, vorzugsweise Cl oder Br, besonders bevorzugt Cl.

In einer weiteren bevorzugten Ausführungsform sind Verbindungen gemäß Formel (IV) bevorzugt, bei denen Z^{b} OR darstellt, wobei R die zuvor, insbesondere für Formel (I) genannte Bedeutung aufweist, wobei Z^{b} bevorzugt eine Alkoxy-Gruppe oder eine Aryloxy-Gruppe mit bevorzugt 1 bis 20 C-Atomen, besonders bevorzugt 1 bis 10 C-Atomen, insbesondere eine Methoxy-, Ethoxy-, Propoxy- oder Phenoxy-Gruppe, vorzugsweise eine Methoxy-, Ethoxy- oder Phenoxy-Gruppe, besonders bevorzugt eine Methoxy- oder Phenoxy-Gruppe ist, und Z^{a} ein Halogen ist, vorzugsweise Cl, Br oder I, vorzugsweise Cl oder Br, besonders bevorzugt Cl.

Erfindungsgemäß wird in Schritt A) eine Kupplungsreaktion durchgeführt, bei der mindestens eine der Gruppen Z^{a}, Z^{b}, Z^{c} bevorzugt zu höchstens 0,5 Mol-%, bevorzugter zu 0,1 Mol-%, besonders bevorzugt zu höchstens 0,05 Mol-% umgesetzt wird.

Geeignet hierfür ist die Kupplungsreaktion, die zu C-C-Verknüpfungen und/oder C-N-Verknüpfungen führt, gemäß SUZUKI. Diese Reaktion ist weithin bekannt, wobei die Beispiele dem Fachmann weitere Hinweise bereitstellen.

Die Grundlagen der zuvor dargelegten Herstellungsverfahren sind im Prinzip aus der Literatur für ähnliche Verbindungen bekannt und können vom Fachmann leicht zur Durchführung der erfindungsgemäßen Verfahren angepasst werden. Weitere Informationen können den Beispielen entnommen werden.

Erfindunggemäß wird im Schritt A) eine Suzuki-Kupplung durchgeführt und vorzugsweise wird mindestens eine der Gruppen Z^{a}, Z^{b}, Z^{c}, die zu höchstens 0,1 Mol-%, bevorzugt zu höchstens 0,05 Mol-% umgesetzt wird, OR ist, wobei R¹ die zuvor, insbesondere für Formel (I) genannte Bedeutung aufweist.

In Schritt A) wird als erste reaktive aromatische oder heteroaromatische Verbindung (A) eine Aryl- oder Heteroarylverbindung der Formel (A) eingesetzt 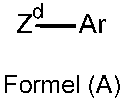
wobei für die verwendeten Symbole gilt:
   - Ar: ist bei jedem Auftreten gleich oder verschieden eine Aryl- oder Heteroarylgruppe mit 5 bis 60 aromatischen Ringatomen, die mit einem oder mehreren Resten R¹ substituiert sein kann;
   - Z^{d}: ist eine reaktive Gruppe B(R^{1a})₂,
   - R^{1a}: ist bei jedem Auftreten gleich oder verschieden OH, eine geradkettige Alkyl- oder Alkoxygruppe mit 1 bis 20 C-Atomen, eine verzweigte oder cyclische Alkyl- oder Alkoxygruppe mit 3 bis 20 C-Atomen, wobei die lineare, verzweigte oder cyclische Alkyl- oder Alkoxygruppe jeweils mit einem oder mehreren Resten R² substituiert sein kann, oder ein aromatisches oder heteroaromatisches Ringsystem mit 5 bis 40 aromatischen Ringatomen, das jeweils durch einen oder mehrere Reste R² substituiert sein kann, dabei können zwei oder mehrere Reste R^{1a} miteinander ein Ringsystem bilden,
wobei R² die zuvor, insbesondere für Formel (I) genannte Bedeutung aufweist.

Die Umsetzung gemäß Schritt A) kann bei einer Temperatur unterhalb von 140°C, vorzugsweise unterhalb von 120°C durchgeführt werden.

Die nach der Umsetzung gemäß Schritt A) erhaltene Zwischenverbindung (ZA) kann vor der weiteren Reaktion gemäß Schritt B) aufgereinigt werden. Hierzu können sämtliche bekannten Aufreinigungsverfahren durchgefügt werden. Vorzugsweise kann nach Schritt A) eine Destillation, eine Chromatographie oder eine Umkristallisation durchgeführt werden, bevor die erhaltene Zwischenverbindung (ZA) umgesetzt wird.

In Schritt B) wird das erhaltene Zwischenprodukts (ZA) mit einer zweiten reaktiven aromatischen oder heteroaromatischen Verbindung (B), die sich von der ersten reaktiven aromatischen oder heteroaromatischen Verbindung (A) unterscheidet, unter Erhalt einer unsymmetrisch substituierten stickstoffhaltigen heterocyclischen Verbindung umgesetzt.

Die erste reaktive aromatische oder heteroaromatische Verbindung (A) unterscheidet sich von der zweiten reaktiven aromatischen oder heteroaromatischen Verbindung (B). Der Unterschied ist in der unsymmetrisch substituierten stickstoffhaltigen heterocyclischen Verbindung sichtbar, die nach der Umsetzung des Zwischenprodukts (ZA) enthalten sind. Somit reicht ein Unterschied in den reaktiven Gruppen der reaktiven Verbindungen (A) und (B) nicht aus, um als Differenz zwischen diesen Verbindungen (A) und (B) zu gelten.

Die in Schritt B) eingesetzte zweite reaktive aromatischen oder heteroaromatischen Verbindung (B) ist eine metallorganische Aryl- oder Heteroarylverbindung.

Insbesondere wird die in Schritt B) als zweite reaktive aromatische oder heteroaromatische Verbindung (B) eine Aryl- oder Heteroarylverbindung der Formel (B) eingesetzt

M-Ar Formel (B)

wobei für die verwendeten Symbole gilt:
   - Ar: ist bei jedem Auftreten gleich oder verschieden eine Aryl- oder Heteroarylgruppe mit 5 bis 60 aromatischen Ringatomen, die mit einem oder mehreren Resten R¹ substituiert sein kann, wobei zwei Reste R auch miteinander ein Ringsystem bilden können;
   - M: ist ein Metallatom, vorzugsweise Li, oder ein metallorganischer Rest, vorzugsweise MgBr oder MgCl,
wobei R¹ die zuvor, insbesondere für Formel (I) genannte Bedeutung aufweist.

In Schritt A) wird vorzugsweise mindestens eine der Gruppen Z^{a}, Z^{b}, Z^{c} zu höchstens 0,5 Mol-%, bevorzugt zu 0,1 Mol-%, besonders bevorzugt zu höchstens 0,05 Mol-% umgesetzt. Die in Schritt A) zu höchstens 0,1 Mol-%, bevorzugt zu höchstens 0,05 Mol-% umgesetzte Gruppe, die ausgewählt ist aus Z^{a}, Z^{b} oder Z^{c}, wird in Schritt B) mit einer metallorganischen Aryl- oder Heteroarylverbindung umgesetzt.

Erfindungsgemäss ist die in Schritt B) umgesetzte Gruppe eine Gruppe der Formel OR, wie diese für Formel (I) dargelegt ist. Bevorzugt kann die in Schritt B) umgesetzte Gruppe eine Alkoxy-Gruppe oder eine Aryloxy-Gruppe mit bevorzugt 1 bis 20 C-Atomen, besonders bevorzugt 1 bis 10 C-Atomen, vorzugsweise eine Methoxy-, Ethoxy-, Propoxy- oder Phenoxy-Gruppe, besonders bevorzugt eine Methoxy-, Ethoxy- oder Phenoxy-Gruppe, speziell bevorzugt eine Methoxy- oder Phenoxy-Gruppe sein.

Die Umsetzung mit einer metallorganischen Aryl- oder Heteroarylverbindung wird ohne den Zusatz von Übergangsmetall-Katalysatoren durchgeführt.

Die Umsetzung gemäß Schritt B) kann bei einer Temperatur unterhalb von 140°C, vorzugsweise unterhalb von 120°C durchgeführt werden.

Die nach der Umsetzung gemäß Schritt B) erhaltene unsymmetrisch substituierte stickstoffhaltige heterocyclische Verbindung kann aufgereinigt werden. Hierzu können sämtliche bekannten Aufreinigungsverfahren durchgefügt werden. Vorzugsweise kann nach Schritt B) eine Destillation, eine Chromatographie oder eine Umkristallisation durchgeführt werden.

Durch diese Verfahren, gegebenenfalls gefolgt von Aufreinigung, wie z. B. Umkristallisation, lassen sich die erfindungsgemäß erhältlichen Zwischenprodukte (ZA) beziehungsweise die unsymmetrisch substituierten stickstoffhaltigen heterocyclischen Verbindungen in hoher Reinheit, bevorzugt mehr als 99 % (bestimmt mittels ¹H-NMR und/oder HPLC) erhalten.

Ein weiterer Gegenstand der vorliegenden Offenbarung ist ein Verfahren zur Herstellung von substituierten stickstoffhaltigen Heterocyclen umfassend die Umsetzung eines Edukts gemäß der folgenden Formel (V), (VI) oder (VII) 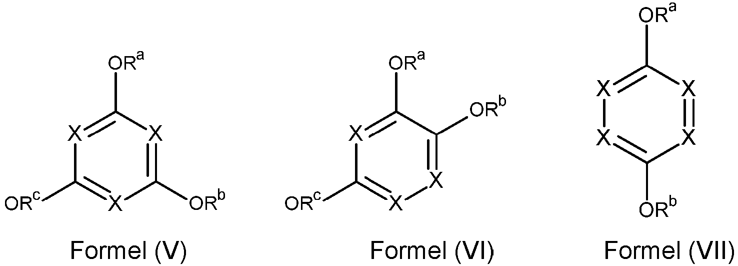
wobei für die verwendeten Symbole und Indizes gilt:
   - R^{a}, R^{b}, R^{c}: ist bei jedem Auftreten gleich oder verschieden N(R²)₂, CN, NO₂, OH, COOH, COOR², C(=O)N(R²)₂, Si(R²)₃, B(OR²)₂, C(=O)R², P(=O)(R²)₂, S(=O)R², S(=O)₂R², OSO₂R², eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R² substituiert sein kann, wobei eine oder mehrere nicht benachbarte CH₂-Gruppen durch R²C=CR², C≡C, Si(R²)₂, C=O, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann, oder eine Aryloxy- oder Heteroaryloxygruppe mit 5 bis 40 aromatischen Ringatomen, die durch einen oder mehrere Reste R² substituiert sein kann; und
   - X und R²: die zuvor, insbesondere für Formel (I) genannten Bedeutungen aufweisen;
mit einer ersten reaktiven aromatischen oder heteroaromatischen Verbindung (A).

In einer ersten Ausführungsform kann vorgesehen sein, dass die Reste R^{a}, R^{b}, R^{c} identisch sind und alle Gruppen OR^{a}, OR^{b}, OR^{c} mit einer ersten reaktiven aromatischen oder heteroaromatischen Verbindung (A) umgesetzt werden. Hierdurch können symmetrisch substituierte stickstoffhaltige heterocyclische Verbindung erhalten werden.

In einer zweiten Ausführungsform kann vorgesehen sein, dass nicht alle der Reste R^{a}, R^{b}, R^{c} identisch sind und nur ein Teil der Gruppen OR^{a}, OR^{b}, OR^{c} mit einer ersten reaktiven aromatischen oder heteroaromatischen Verbindung (A) unter Erhalt eines Zwischenprodukts (ZA) umgesetzt werden. Das Zwischenprodukt (ZA) kann mit einer zweiten reaktiven aromatischen oder heteroaromatischen Verbindung (B) umgesetzt werden, die sich von der ersten reaktiven aromatischen oder heteroaromatischen Verbindung (A) unterscheidet, unter Erhalt einer unsymmetrisch substituierten stickstoffhaltigen heterocyclischen Verbindung.

Die für die Umsetzung mit Edukten gemäß den Formeln (V), (VI) oder (VII) vorgesehenen bevorzugten reaktiven aromatischen oder heteroaromatischen Verbindungen (A) und (B) wurden zuvor im Zusammenhang mit der Umsetzung der Edukte gemäß den Formeln (I), (II), (III) oder (IV) beschrieben.

Hierbei sind insbesondere Aryl- oder Heteroarylverbindungen der zuvor dargelegten Formel (B) bevorzugt, wobei die hierfür dargelegten bevorzugten Reaktionsbedingungen, beispielsweise der Verzicht auf den Zusatz von Übergangsmetall-Katalysatoren, auch für diese Umsetzungen bevorzugt sind.

Ferner kann vorgesehen sein, dass die Gruppe Ar in Formel (A) und/oder Formel (B) ausgewählt ist aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 9,9`-Diaryl-Fluorenyl 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl, Pyrenyl, Triazinyl, Imidazolyl, Benzimidazolyl, Benzoxazolyl, Benzthiazolyl, 1-, 2-, 3- oder 4-Carbazolyl, 1- oder 2-Napthyl, Anthracenyl, vorzugsweise 9-Anthracenyl, Trans- und cis-Indenofluorenyl, Indenocarbazolyl, Indolocarbazolyl, Spirocarbazolyl, 5-Aryl-Phenanthridin-6-on-yl, 9,10-Dehydrophenanthrenyl, Fluoranthenyl, Tolyl, Mesityl, Phenoxytolulyl, Anisolyl, Triarylaminyl, Bis-triarylaminyl, Tristriarylaminyl, Hexamethylindanyl, Tetralinyl, Monocycloalkyl, Biscycloalkyl, Tricycloalkyl, Alkyl, wie z.B. tert-Butyl, Methyl, Propyl, Alkoxyl, Alkylsulfanyl, Alkylaryl, Triarylsilyl, Trialkylsilyl, Xanthenyl, 10-Aryl-Phenoxazinyl, Phenanthrenyl und/oder Triphenylenyl, die jeweils durch einen oder mehrere Reste substituiert sein können, bevorzugt aber unsubstituiert sind, wobei Phenyl, Spirobifluoren-, Fluoren-, Dibenzofuran-, Dibenzothiophen-, Anthracen-, Phenanthren-, Triphenylen- Gruppen besonders bevorzugt sind. Hierbei können die zuvor dargelegten Gruppen durch Gruppen R¹ substituiert sein, wie diese zuvor beschrieben wurden.

Bevorzugt kann die Gruppe Ar in Formel (A) und/oder Formel (B) ausgewählt sein aus der Gruppe der Fluorene, Indenofluorene, Spirobifluorene, Carbazole, Indenocarbazole, Indolocarbazole, Spirocarbazole, Pyrimidine, Triazine, Lactame, Triarylamine, Dibenzofurane, Dibenzothiene, Imidazole, Benzimidazole, Benzoxazole, Benzthiazole, 5-Aryl-Phenanthridin-6-one, 9,10-Dehydrophenanthrene, Fluoranthene, wobei die Gruppe mit einem oder mehreren Restens R¹ substituiert sein kann.

Wenn zwei Reste R¹ bzw. R² bzw. R³ miteinander ein Ringsystem bilden, so kann dieses mono- oder polycyclisch, aliphatisch, heteroaliphatisch, aromatisch oder heteroaromatisch sein. Dabei können die Reste, die miteinander ein Ringsystem bilden, benachbart sein, d.h. dass diese Reste an dasselbe Kohlenstoffatom oder an Kohlenstoffatome, die direkt aneinander gebunden sind, gebunden sind, oder sie können weiter voneinander entfernt sein.

Weiterhin kann vorgesehen sein, dass die Substitutenten R¹ mit Ringatomen eines aromatischen oder heteroaromatischen Ringssystems, an das die Substitutenten R¹ binden, kein kondensiertes aromatisches oder heteroaromatisches Ringsystem, vorzugsweise kein kondensiertes Ringsystem bilden. Dies schließt die Bildung eines kondensierten Ringsystems mit möglichen Substituenten R², R³ ein, die an die Reste R¹ gebunden sein können. Bevorzugt kann vorgesehen sein, dass die Substitutenten R¹ eines aromatischen oder heteroaromatischen Ringssystems mit den Ringatomen des aromatischen oder heteroaromatischen Ringssystems kein Ringsystem bilden. Dies schließt die Bildung eines Ringsystems mit möglichen Substituenten R², R³ ein, die an die Reste R¹ gebunden sein können.

Wenn Z^{a}, Z^{b}, Z^{c} für OR steht bzw. wenn die aromatische und/oder heteroaromatische Gruppen einen Rest R^{a}, R^{b}, R^{c} aufweisen, dann sind diese Reste R, R^{a}, R^{b}, R^{c} bevorzugt gewählt aus der Gruppe bestehend aus F, CN, N(Ar¹)₂, C(=O)Ar¹, P(=O)(Ar¹)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R² substituiert sein kann, wobei eine oder mehrere nichtbenachbarte CH₂-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten R² substituiert sein kann, bevorzugt aber unsubstituiert ist, oder einer Aralkyl- oder Heteroaralkylgruppe mit 5 bis 25 aromatischen Ringatomen, die mit einem oder mehreren Resten R² substituiert sein kann; wobei Ar¹ gleich oder verschieden bei jedem Auftreten ein aromatisches oder heteroaromatisches Ringsystem mit 5 bis 40 aromatischen Ringatomen, das jeweils mit einem oder mehreren Resten R² substituiert sein kann, eine Aryloxygruppe mit 5 bis 40 aromatischen Ringatomen, die mit einem oder mehreren Resten R² substituiert sein kann, oder einer Aralkylgruppe mit 5 bis 40 aromatischen Ringatomen, die jeweils mit einem oder mehreren Resten R² substituiert sein kann, darstellt, wobei optional zwei oder mehr, vorzugsweise benachbarte Substituenten R² ein mono- oder polycyclisches, aliphatisches, heteroaliphatisches, aromatisches oder heteroaromatisches Ringsystem vorzugsweise ein mono- oder polycyclisches, aliphatisches Ringsystem bilden können, das mit einem oder mehreren Resten R³ substituiert sein kann, wobei das Symbol R² die zuvor, insbesondere für Formel (I) genannte Bedeutung aufweisen kann.

Vorzugsweise stellt Ar¹ gleich oder verschieden bei jedem Auftreten eine Aryl- oder Heteroarylgruppe mit 5 bis 24, vorzugsweise 5 bis 12 aromatischen Ringatomen dar, die jeweils mit einem oder mehreren Resten R² substituiert sein kann, vorzugsweise jedoch unsubstituiert ist.

Beispiele für geeignete Gruppen Ar¹ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste R² substituiert sein können, bevorzugt aber unsubstituiert sind.

Besonders bevorzugt sind diese Reste R, R^{a}, R^{b}, R^{c} ausgewählt aus der Gruppe bestehend aus H, D, F, CN, N(Ar¹)₂, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevorzugt mit 2, 3 oder 4 C-Atomen, die jeweils mit einem oder mehreren Resten R² substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten R² substituiert sein kann, bevorzugt aber unsubstituiert ist.

Wenn X für CR¹ steht bzw. wenn die aromatische und/oder heteroaromatische Gruppen durch Substituenten R¹ substituiert sind, dann sind diese Substituenten R¹ bevorzugt gewählt aus der Gruppe bestehend aus H, D, F, CN, N(Ar¹)₂, C(=O)Ar¹, P(=O)(Ar¹)₂, einer geradkettigen Alkyl- oder Alkoxygruppe mit 1 bis 10 C-Atomen oder einer verzweigten oder cyclischen Alkyl- oder Alkoxygruppe mit 3 bis 10 C-Atomen oder einer Alkenylgruppe mit 2 bis 10 C-Atomen, die jeweils mit einem oder mehreren Resten R² substituiert sein kann, wobei eine oder mehrere nichtbenachbarte CH₂-Gruppen durch O ersetzt sein können und wobei ein oder mehrere H-Atome durch D oder F ersetzt sein können, einem aromatischen oder heteroaromatischen Ringsystem mit 5 bis 24 aromatischen Ringatomen, das jeweils mit einem oder mehreren Resten R² substituiert sein kann, bevorzugt aber unsubstituiert ist, oder einer Aralkyl- oder Heteroaralkylgruppe mit 5 bis 25 aromatischen Ringatomen, die mit einem oder mehreren Resten R² substituiert sein kann; dabei können optional zwei Substituenten R¹, die an dasselbe Kohlenstoffatom oder an benachbarte Kohlenstoffatome gebunden sind, ein monocyclisches oder polycyclisches, aliphatisches, aromatisches oder heteroaromatisches Ringsystem bilden, das mit einem oder mehreren Resten R² substituiert sein kann; wobei Ar¹ die zuvor genannte Bedeutung aufweist.

Besonders bevorzugt sind diese Substituenten R¹ ausgewählt aus der Gruppe bestehend aus H, D, F, CN, N(Ar¹)₂, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3 oder 4 C-Atomen, oder einer Alkenylgruppe mit 2 bis 8 C-Atomen, bevorzugt mit 2, 3 oder 4 C-Atomen, die jeweils mit einem oder mehreren Resten R² substituiert sein kann, bevorzugt aber unsubstituiert ist, oder einem aromatischen oder heteroaromatischen Ringsystem mit 6 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten R² substituiert sein kann, bevorzugt aber unsubstituiert ist; dabei können optional zwei Substituenten R¹, die an dasselbe Kohlenstoffatom oder an benachbarte Kohlenstoffatome gebunden sind, ein monocyclisches oder polycyclisches, aliphatisches Ringsystem bilden, das mit einem oder mehreren Resten R² substituiert sein kann, bevorzugt aber unsubstituiert ist, wobei Ar¹ die zuvor dargelegte Bedeutung aufweisen kann.

Ganz besonders bevorzugt sind die Substituenten R¹ ausgewählt aus der Gruppe bestehend aus einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten R² substituiert sein kann, bevorzugt aber unsubstituiert ist. Beispiele für geeignete Substituenten R¹ sind ausgewählt aus der Gruppe bestehend aus Phenyl, ortho-, meta- oder para-Biphenyl, Terphenyl, insbesondere verzweigtes Terphenyl, Quaterphenyl, insbesondere verzweigtes Quaterphenyl, 1-, 2-, 3- oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3- oder 4-Dibenzothienyl und 1-, 2-, 3- oder 4-Carbazolyl, die jeweils durch einen oder mehrere Reste R² substituiert sein können, bevorzugt aber unsubstituiert sind.

Weiterhin kann vorgesehen sein, dass die Reste R, R^{a}, R^{b}, R^{c} gemäß Formeln (I), (IV), , die Gruppen Ar der Verbindungen gemäß Formel (A) oder (B) und/oder einer der Substituenten R¹ eine Gruppe umfasst, vorzugsweise für eine Gruppe steht, die ausgewählt ist aus den Formeln (R¹-1) bis bis (R¹- 95) 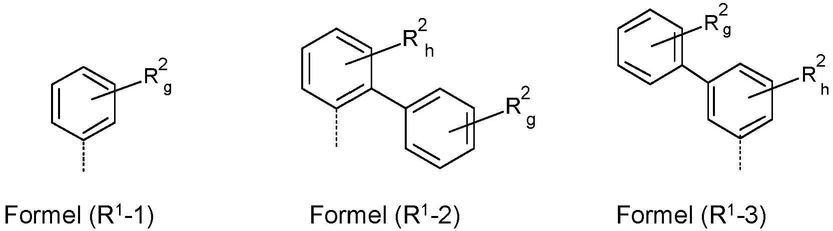 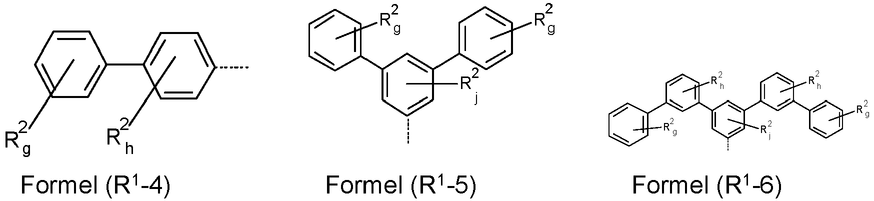 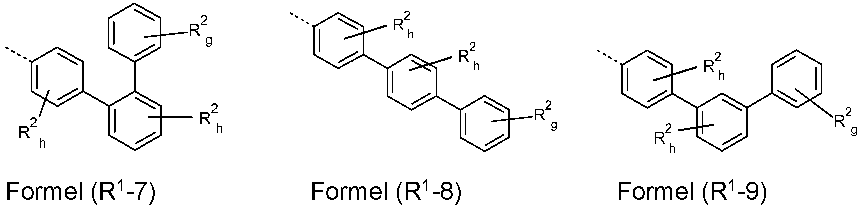 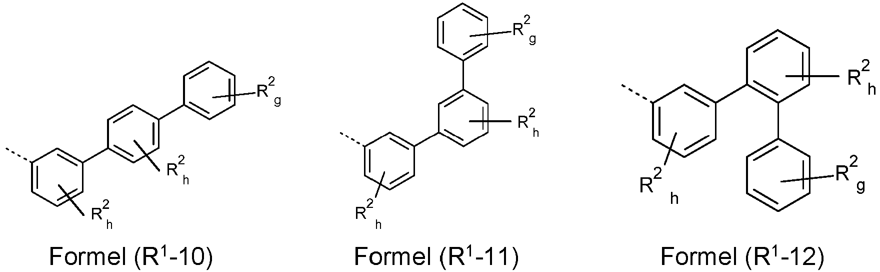 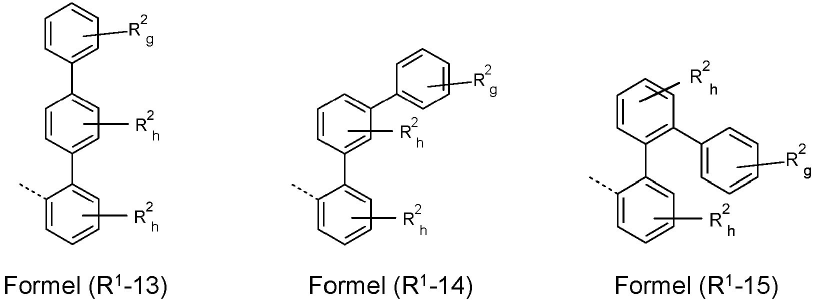 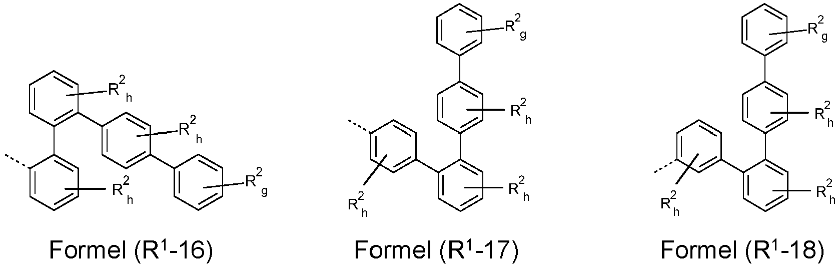 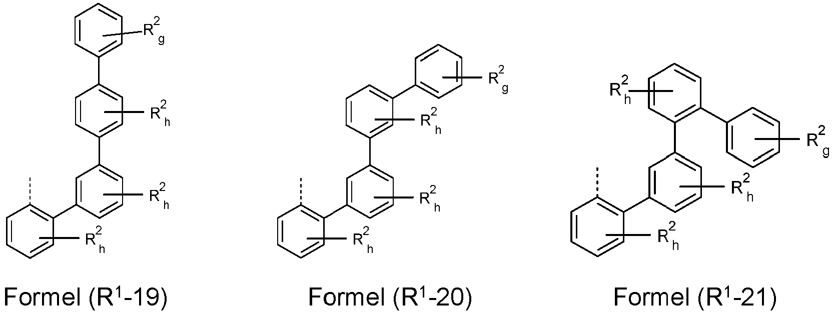 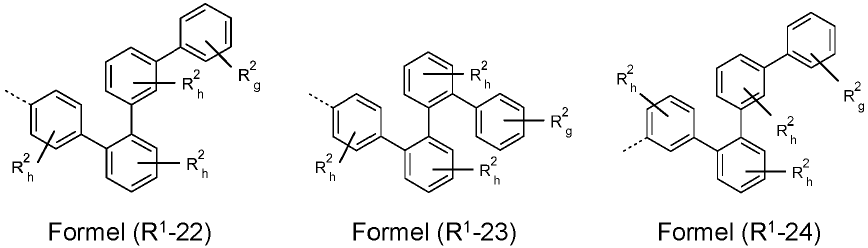 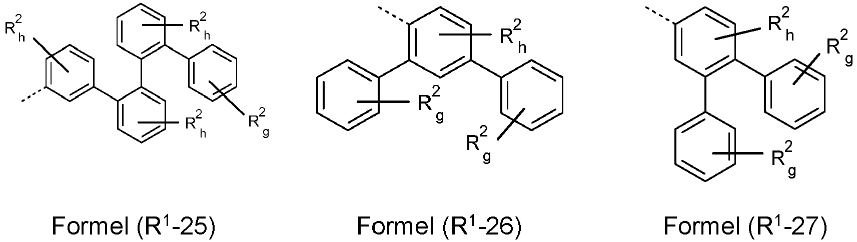 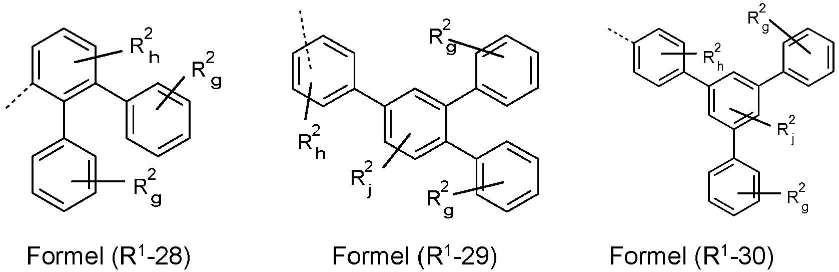 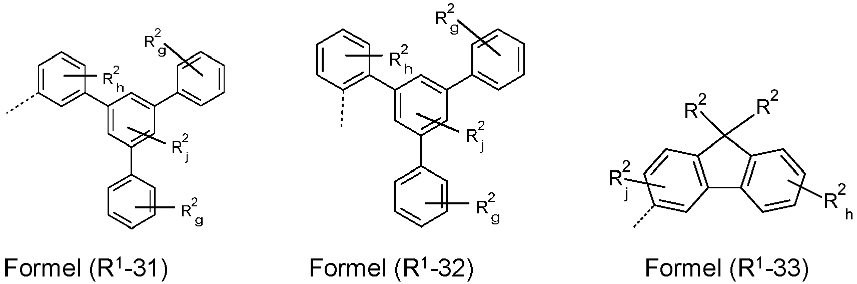 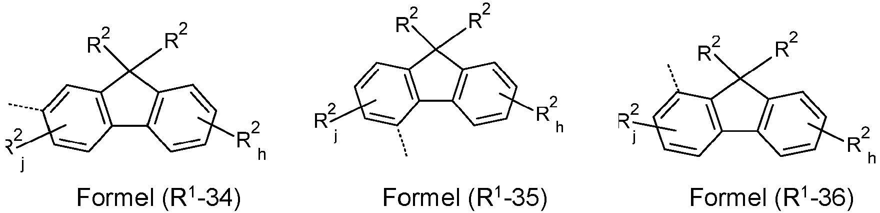 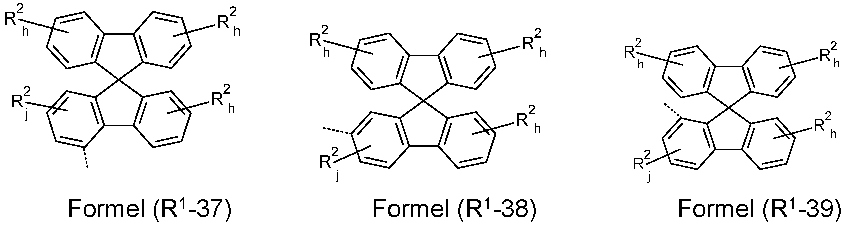 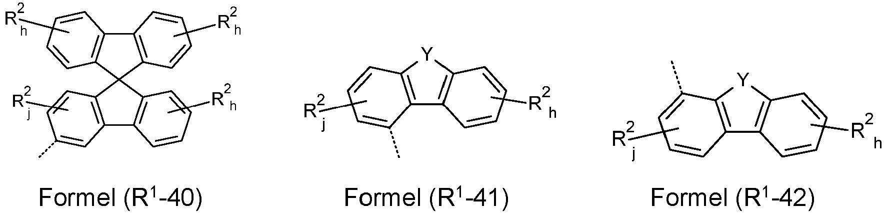 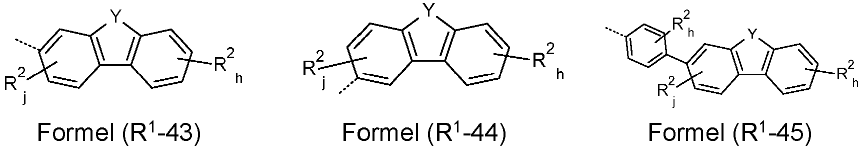 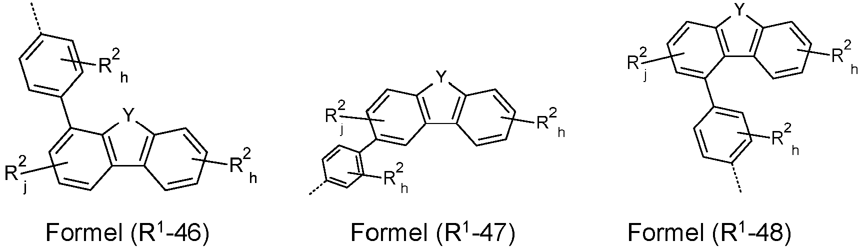 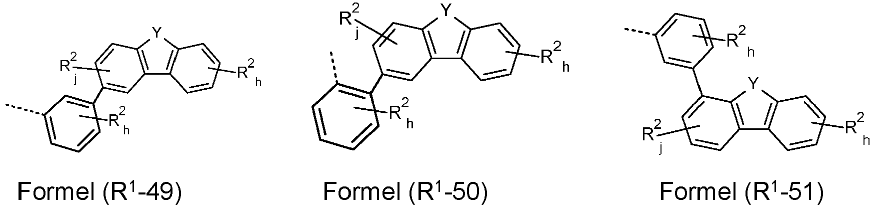 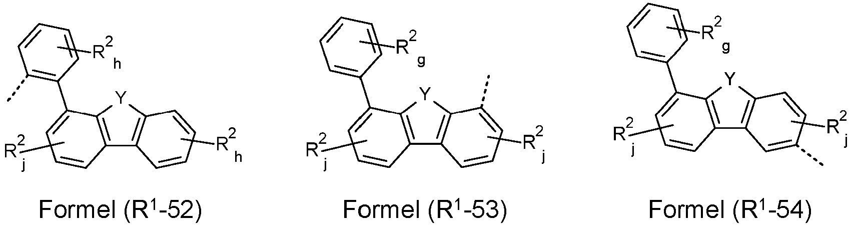 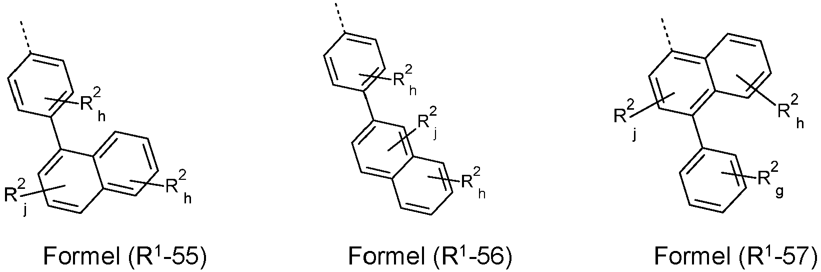 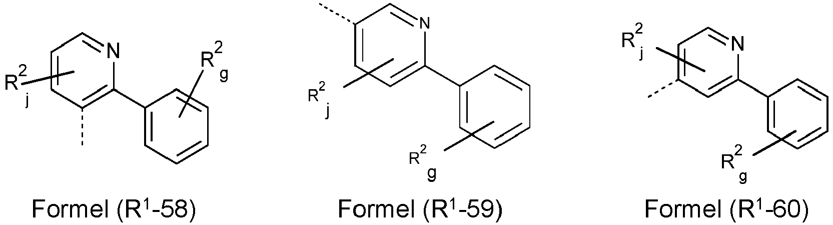 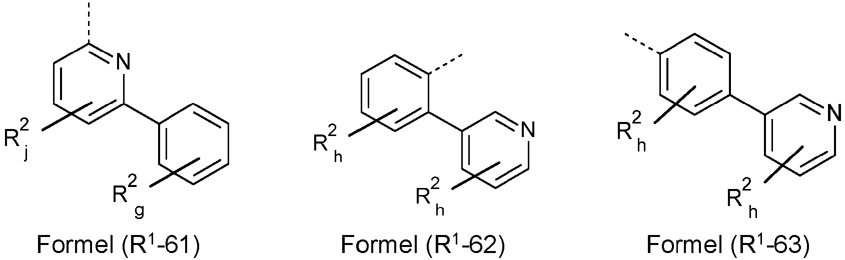 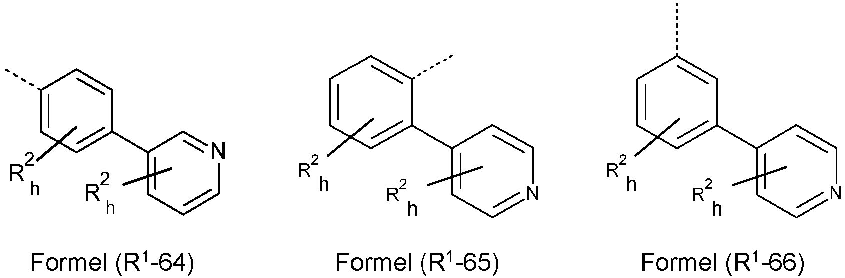 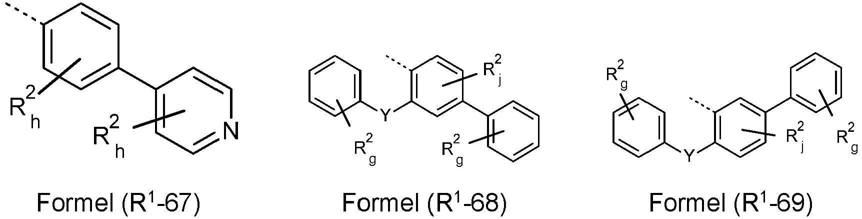 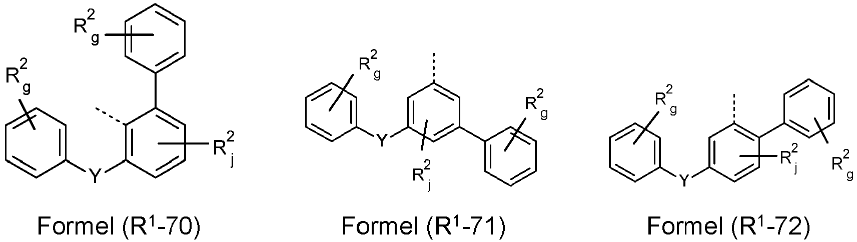 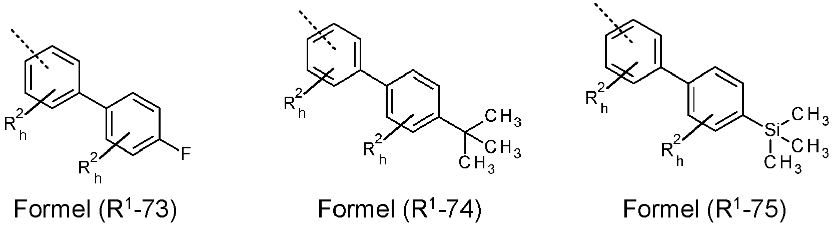 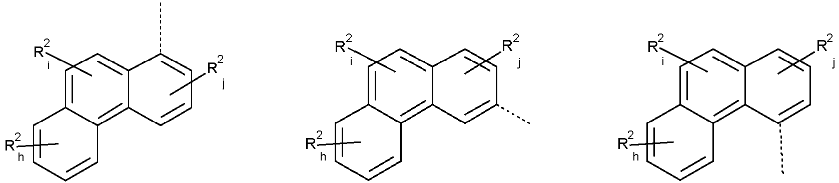 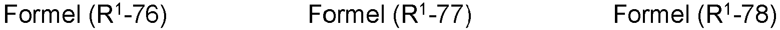 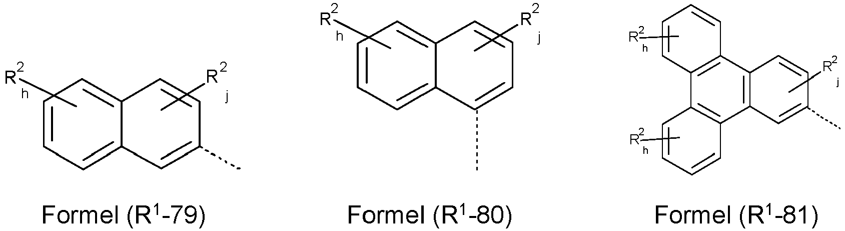 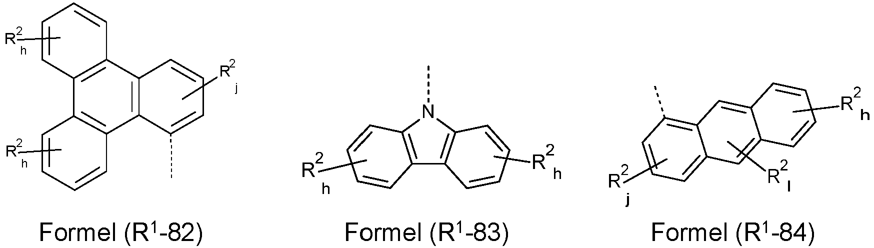 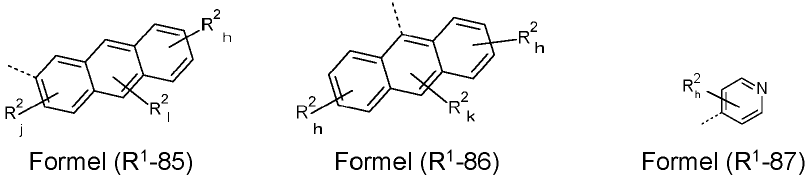 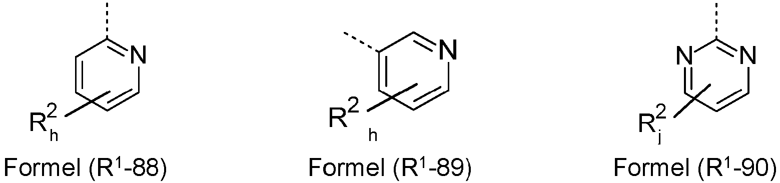 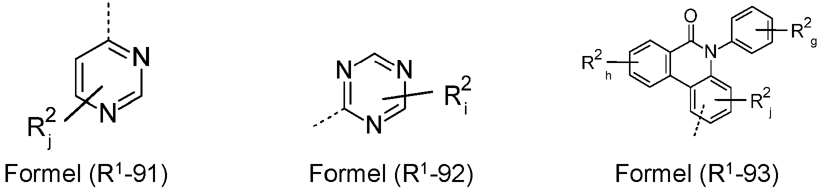 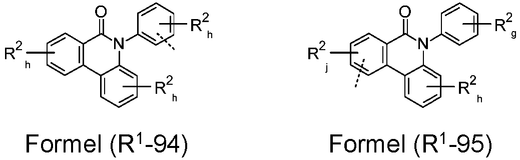
wobei für die verwendeten Symbole gilt:
   - Y: ist O, S oder NR², vorzugsweise O oder S;
   - i: ist bei jedem Auftreten unabhängig 0, 1 oder 2;
   - j: ist bei jedem Auftreten unabhängig 0, 1, 2 oder 3;
   - h: ist bei jedem Auftreten unabhängig 0, 1, 2, 3 oder 4;
   - g: ist bei jedem Auftreten unabhängig 0, 1, 2, 3, 4 oder 5;
   - R²: kann die zuvor genannte, insbesondere für Formel (I) genannte Bedeutung aufweisen und
die gestrichelte Bindung markiert die Anbindungsposition.

Vorzugsweise kann vorgesehen sein, dass die Summe der Indices i, j, h und g in den Strukturen der Formel (R¹-1) bis (R¹-95) jeweils höchstens 3, vorzugsweise höchstens 2 und besonders bevorzugt höchstens 1 beträgt.

Bevorzugt bilden die Reste R² in den Formeln (R¹-1) bis (R¹-95) mit den Ringatomen der Arylgruppe oder Heteroarylgruppe, an die die Reste R² gebunden sind, kein kondensiertes aromatisches oder heteroaromatisches Ringsystem, vorzugsweise kein kondensiertes Ringsystem. Dies schließt die Bildung eines kondensierten Ringsystems mit möglichen Substituenten R³ ein, die an die Reste R² gebunden sein können.

Der Rest Ar gemäß Formel (A) oder (B) ist mit Resten R¹ substituiert. Bevorzugte Ausführungsformen des Rests Ar gemäß Formel (A) oder (B) ergeben sich aus den Strukturen der Formeln (R¹-1) bis (R¹-95), wobei in diesem Fall die Substituenten R² in den Formeln (R¹-1) bis (R¹-95) durch Substituenten R¹ zu ersetzen sind. Die zuvor dargelegten Bevorzugungen hinsichtlich der Formeln (R¹-1) bis (R¹-95) gelten entsprechend.

In einer weiteren bevorzugten Ausführungsform der Erfindung ist R², beispielsweise in einer Struktur gemäß Formeln (I), (IV), in einer Struktur der Verbindungen gemäß Formel (A) oder (B) und/oder bei einer Substitution eines Rests R¹ sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

In einer weiteren bevorzugten Ausführungsform der Erfindung ist R³, beispielsweise in einer Struktur gemäß Formeln (I), (IV), , in einer Struktur der Verbindungen gemäß Formel (A) oder (B) und/oder bei einer Substitution eines Rests R² sowie bevorzugten Ausführungsformen dieser Struktur oder den Strukturen, bei denen Bezug auf diese Formeln genommen wird, bei jedem Auftreten gleich oder verschieden ausgewählt aus der Gruppe bestehend aus H, D, F, CN, einem aliphatischen Kohlenwasserstoffrest mit 1 bis 10 C-Atomen, bevorzugt mit 1, 2, 3 oder 4 C-Atomen, oder einem aromatischen oder heteroaromatischen Ringsystem mit 5 bis 30 aromatischen Ringatomen, bevorzugt mit 5 bis 24 aromatischen Ringatome, besonders bevorzugt mit 5 bis 13 aromatischen Ringatomen, das durch ein oder mehrere Alkylgruppen mit jeweils 1 bis 4 Kohlenstoffatomen substituiert sein kann, bevorzugt aber unsubstituiert ist.

Gemäß einer bevorzugten Ausführungsform der vorliegenden Erfindung kann vorgesehen sein, dass ein funktionales Material (OSM) hergestellt wird, welches zur Herstellung von Funktionsschichten elektronischer Vorrichtungen einsetzbar ist, welches vorzugsweise ausgewählt ist aus der Gruppe bestehend aus fluoreszierenden Emittern, phosphoreszierenden Emittern, Emittern, die TADF (thermally activated delayed fluorescence) zeigen, Hostmaterialien, Elektronentransportmaterialien, Excitonenblockiermaterialien, Elektroneninjektionsmaterialien, Lochleitermaterialien, Lochinjektionsmaterialien, n-Dotanden, p-Dotanden, Wide-Band-Gap-Materialien, Elektronenblockiermaterialien und/oder Lochblockiermaterialien. Diese funktionalen Materialien (OSM) sind in der Fachwelt weithin bekannt und beispielsweise in WO 2011/076314 A1, WO 2016/107663 A1 und WO 2017/036572 A1 offenbart, wobei diese Dokumente durch Referenz hierauf in die vorliegende Anmeldung aufgenommen werden.

Bevorzugt kann gemäß dem Verfahren, bei dem ein substituierter stickstoffhaltiger Heterocyclus hergestellt wird, die erste reaktive aromatische oder heteroaromatische Verbindung (A) oder die zweite reaktiven aromatischen oder heteroaromatischen Verbindung (B) ausgewählt sein aus der Gruppe bestehend aus der Gruppe der Fluorene, Indenofluorene, Spirobifluorene, Carbazole, Indenocarbazole, Indolocarbazole, Spirocarbazole, Pyrimidine, Triazine, Lactame, Triarylamine, Dibenzofurane, Dibenzothiene, Imidazole, Benzimidazole, Benzoxazole, Benzthiazole, 5-Aryl-Phenanthridin-6-one, 9,10-Dehydrophenanthrene, Fluoranthene.

Das Verfahren der vorliegenden Erfindung dient unter anderem zur Herstellung von einem oganisch funktionalem Material (OSM). Ferner kann eine gemäß dem vorliegenden Verfahren erhältliche Verbindung in Kombination mit einem weiteren organisch funktionalen Material eingesetzt werden.

Die erfindungsgemäßen Verfahren zeichnen sich durch einen oder mehrere der folgenden überraschenden Vorteile gegenüber dem Stand der Technik aus:
1. Die erfindungsgemäßen Verfahren ermöglichen eine einfache und kostengünstige Herstellung von substituierten stickstoffhaltigen Heterocyclen, die sich insbesondere zur Herstellung von elektronischen Vorrichtungen eignen.
2. Die erfindungsgemäß erhältlichen stickstoffhaltigen Heterocyclen lassen sich in sehr hoher Ausbeute und sehr hoher Reinheit bei außergewöhnlich kurzen Reaktionszeiten und vergleichsweise geringen Reaktionstemperaturen durch die erfindungsgemäßen Verfahren synthetisieren. Hierbei werden bei den einzelnen Verfahrensschritten erstaunlich hohe Selektivitäten erzielt. Weiterhin kann vielfach auf eine aufwändige Reinigung der Zwischen- oder Endprodukte verzichtet werden.
3. Die erfindungsgemäßen Verfahren können mit bekannten Vorrichtungen ausgeführt werden, so dass hierdurch eine hohe Kosteneffizienz erzielt werden kann.

Diese oben genannten Vorteile gehen nicht mit einer Verschlechterung der weiteren elektronischen Eigenschaften einher.

Die erfindungsgemäß erhältlichen stickstoffhaltigen Heterocyclen eignen sich für die Verwendung in einer elektronischen Vorrichtung. Dabei wird unter einer elektronischen Vorrichtung eine Vorrichtung verstanden, welche mindestens eine Schicht enthält, die mindestens eine organische Verbindung enthält. Das Bauteil kann dabei aber auch anorganische Materialien enthalten oder auch Schichten, welche vollständig aus anorganischen Materialien aufgebaut sind.

Es sei darauf hingewiesen, dass Variationen der in der vorliegenden Erfindung beschriebenen Ausführungsformen unter den Umfang dieser Erfindung fallen. Jedes in der vorliegenden Erfindung offenbarte Merkmal kann, sofern dies nicht explizit ausgeschlossen wird, durch alternative Merkmale, die demselben, einem äquivalenten oder einem ähnlichen Zweck dienen, ausgetauscht werden. Somit ist jedes in der vorliegenden Erfindung offenbartes Merkmal, sofern nichts anderes gesagt wurde, als Beispiel einer generischen Reihe oder als äquivalentes oder ähnliches Merkmal zu betrachten.

Alle Merkmale der vorliegenden Erfindung können in jeder Art miteinander kombiniert werden, es sei denn dass sich bestimmte Merkmale und/oder Schritte sich gegenseitig ausschließen. Dies gilt insbesondere für bevorzugte Merkmale der vorliegenden Erfindung. Gleichermaßen können Merkmale nicht wesentlicher Kombinationen separat verwendet werden (und nicht in Kombination).

Es sei ferner darauf hingewiesen, dass viele der Merkmale, und insbesondere die der bevorzugten Ausführungsformen der vorliegenden Erfindung selbst erfinderisch und nicht lediglich als Teil der Ausführungsformen der vorliegenden Erfindung zu betrachten sind. Für diese Merkmale kann ein unabhängiger Schutz zusätzlich oder alternativ zu jeder gegenwärtig beanspruchten Erfindung begehrt werden.

Die mit der vorliegenden Erfindung offengelegte Lehre zum technischen Handeln kann abstrahiert und mit anderen Beispielen kombiniert werden.

Die Erfindung wird durch die nachfolgenden Beispiele näher erläutert, ohne sie dadurch einschränken zu wollen.

Der Fachmann kann aus den Schilderungen ohne erfinderisches Zutun weitere erfindungsgemäße elektronische Vorrichtungen herstellen und somit die Erfindung im gesamten beanspruchten Bereich ausführen.

### Beispiele

Die nachfolgenden Synthesen werden, sofern nicht anders angegeben, unter einer Schutzgasatmosphäre in getrockneten Lösungsmitteln durchgeführt. Die Edukte können von ALDRICH bezogen werden.

### Synthesebeispiele

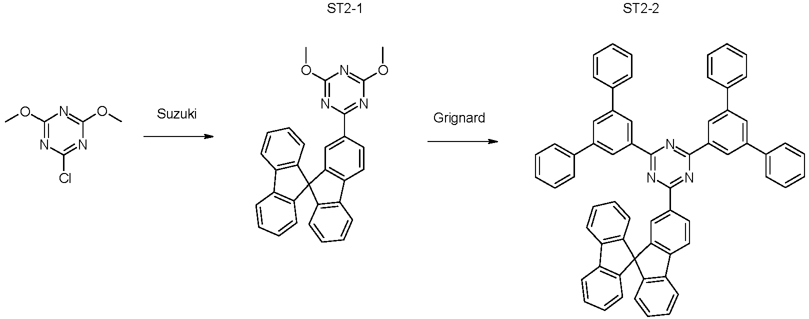

### ST2-1:

2-Chlor-4,6-dimethoxy-1,3,5-triazin (100 mol%), 9,9'-Spirobifluoren-2-yl-boronsäure (110 mol%) und tri-Kaliumphosphat-Trihydrat (120 mol%) werden in einer Apparatur vorgelegt. Die Feststoffe werden dann in THF (1114 mol%) und VE-Wasser (5553 mol%) unter Rühren suspendiert. Anschließend wird [1,1'-Bis(di-phenylphosphino)ferrocene]-dichlorpalladium-(II) (0,5 mol%) zugegeben. Das Reaktionsgemisch wird für 12 h unter Rückfluss erhitzt.

Das organische Lösemittel wird im Vakuum abdestilliert. Zu der erhaltenen Suspension wird 2-Propanol zugegeben und die erhaltenen Kristalle werden isoliert und mit 2-Propanol gewaschen. Das Rohprodukt wird in Xylol gelöst und warm über Kieselgel filtriert. Die erhaltene Lösung wird eingeengt und die erhaltenen Kristalle werden isoliert.

### Ausbeute: 85%, Reinheit >99%

### ST2-2:

### Herstellung Grignard-Reagenz:

Bis-(m-phenyl)-phenyl-bromid (345 mol%) wird in Tetrahydrofuran (2868 mol%) klar gelöst (Bromid-Lsg.). Dann wird in einer inertisierten Apparatur Magnesium (350 mol%) in THF (320 mol%) suspendiert. Nach Zugabe von ca. 10% der Bromid-Lösung wird die Reaktionsmischung vorsichtig erwärmt. Am Rückfluss wird die restliche Bromid-Lsg. zudosiert. Das Reaktionsgemisch wird für weitere 2 h am Rückfluss erhitzt und anschließend abgekühlt. Die so erhaltenen Grignard-Lösung wird im Folgenden weiter eingesetzt.

### Substitution:

In einer weiteren Apparatur wird das ST2-1 (100 mol%) in Tetrahydrofuran (5900 mol%) unter Rühren suspendiert. Dieser Suspension wird dann bei 20-40 °C das Grignard-Reagenz zugegeben. Anschließend wird das Reaktionsgemisch auf 60 °C erhitzt und 15-18 h nachgerührt.

Nach dem Abkühlen wird Essigsäure (30%ig) (380 mol%, exotherm!) und VE-Wasser zugegeben. THF wird im Vakuum abdestilliert und anschließend wird Aceton zugegeben. Die erhaltenen Kristalle werden abgetrennt. Das Rohprodukt wird in Chlorbenzol in der Hitze gelöst und noch warm über AlOx filtriert. Nach dem Einengen bilden sich Kristalle, die dann isoliert werden

Diese Kristalle werden ein weiteres Mal aus Chlorbenzol umkristallisiert. Nach Sublimation erhält man 65% Ausbeute bei >99,9% Reinheit

Über 2 Stufen inkl Sublimation wird eine von Ausbeute 56% erhalten.

Die Verfahren aus dem Stand der Technik ergeben eine Ausbeute von höchstens 43% Ausbeute, wobei diese Verfahren aufwendiger sind und eine verlustreiche Aufarbeitung notwendig ist, da die erste Stufe nicht selektiv ist (vgl. WO 2010/072300, Beispiele 1, 2 und 3).

Das erfindungsgemäße Verfahren stellt demgemäß deutliche Vorteile gegenüber bekannten Methoden bereit, da Sie bessere Ausbeuten und höhere (Roh-)Reinheiten erreicht und die Methoxy-Substitution nichtkatalysiert stattfindet.

Weitere Beispiele werden tabellarisch genannt. Dem Fachmann ist durch oben geschilderte Vorgehensweise ohne weitere erfinderische Tätigkeit die Herstellung der weiteren Beispiele möglich, wobei die gestrichelten Linien die Anbindungsstellen zu den genannten reaktiven Gruppen darstellen.

| | Formel (A) | Formel (B) | Ausbeute |
|---|---|---|---|
| 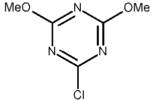 | 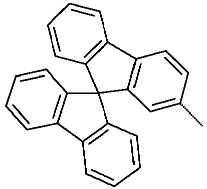 | 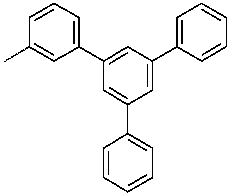 | 41% |
| 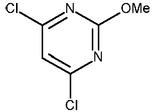 | 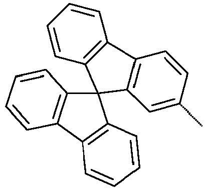 | 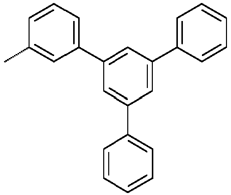 | 51% |
| 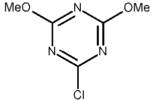 | 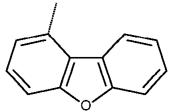 | 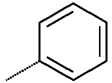 | 61% |
| 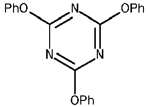 | 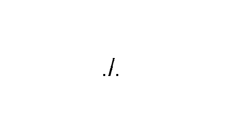 | 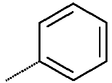 | 57% |
| 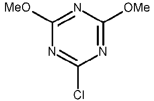 | 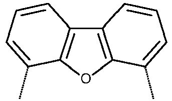 | 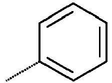 | 44% |
| 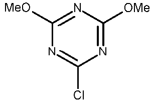 | 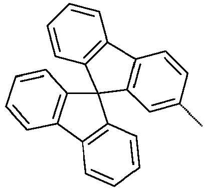 | 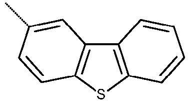 | 47% |
| 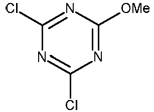 | 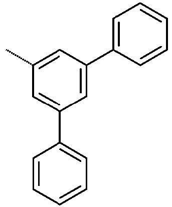 | 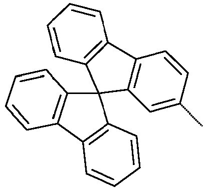 | 54% |
| 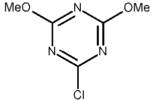 | 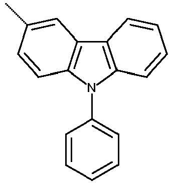 | 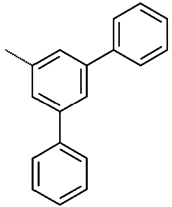 | 54% |

## Patentansprüche

1. Verfahren zur Herstellung von unsymmetrisch substituierten stickstoffhaltigen Heterocyclen umfassend die Schritte:
A) Umsetzung eines Edukts gemäß der folgenden Formel (I) 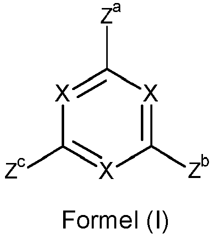
wobei für die verwendeten Symbole und Indizes gilt:
Z^{a}, Z^{b}, Z^{c} ist Halogen oder OR;
X ist bei jedem Auftreten gleich oder verschieden CR¹ oder N, vorzugsweise N, mit der Maßgabe, dass mindestens zwei der Symbole X für N und speziell bevorzugt alle Symbole X für N stehen;
R, ist bei jedem Auftreten gleich oder verschieden, S(=O)₂R², eine geradkettige Alkylgruppe mit 1 bis 20 C-Atomen, eine verzweigte oder cyclische Alkylgruppe mit 3 bis 20 C-Atomen, wobei die lineare, verzweigte oder cyclische Alkylgruppe jeweils mit einem oder mehreren Resten R² substituiert sein kann, oder ein aromatisches oder heteroaromatisches Ringsystem mit 5 bis 40 aromatischen Ringatomen, das jeweils durch einen oder mehrere Reste R² substituiert sein kann,;
R¹ ist bei jedem Auftreten gleich oder verschieden eine geradkettige Alkylgruppe mit 1 bis 20 C-Atomen, eine verzweigte oder cyclische Alkylgruppe mit 3 bis 20 C-Atomen, wobei die lineare, verzweigte oder cyclische Alkylgruppe jeweils mit einem oder mehreren Resten R² substituiert sein kann, oder ein aromatisches oder heteroaromatisches Ringsystem mit 5 bis 40 aromatischen Ringatomen, das jeweils durch einen oder mehrere Reste R² substituiert sein kann, dabei können zwei oder mehrere Reste R¹ miteinander ein Ringsystem bilden;
R² ist bei jedem Auftreten gleich oder verschieden H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R³ substituiert sein kann, wobei eine oder mehrere nicht benachbarte CH₂-Gruppen durch R³C=CR³, C≡C, Si(R³)₂, C=O, NR³, O, S oder CONR³ ersetzt sein können, oder ein aromatisches oder heteroaromatisches Ringsystem mit 5 bis 40 aromatischen Ringatomen, das jeweils durch einen oder mehrere Reste R³ substituiert sein kann, oder eine Aryloxy- oder Heteroaryloxygruppe mit 5 bis 40 aromatischen Ringatomen, die durch einen oder mehrere Reste R³ substituiert sein kann, oder eine Aralkyl- oder Heteroaralkylgruppe mit 5 bis 40 aromatischen Ringatomen, die durch einen oder mehrere Reste R³ substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R³ substituiert sein kann; dabei können zwei oder mehrere Reste R² miteinander ein Ringsystem bilden;
R³ ist bei jedem Auftreten gleich oder verschieden H, D, F oder ein aliphatischer, aromatischer und/oder heteroaromatischer organischer Rest, insbesondere ein Kohlenwasserstoffrest, mit 1 bis 20 C-Atomen, in dem auch ein oder mehrere H-Atome durch F ersetzt sein können, dabei können zwei oder mehrere Substituenten R³ auch miteinander ein mono- oder polycyclisches Ringsystem bilden;
mit der Maßgabe, dass die Gruppe Z^{a} ungleich der Gruppe Z^{b} oder Z^{c} und mindestens eine der Gruppen Z^{a}, Z^{b}, Z^{c} ein Halogen ist;
mit einer ersten reaktiven aromatischen oder heteroaromatischen Verbindung (A) unter Erhalt eines Zwischenprodukts (ZA), wobei die erste reaktive aromatische oder heteroaromatische Verbindung (A) eine Aryl- oder Heteroarylverbindung der Formel (A) ist 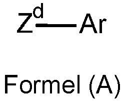 wobei für die verwendeten Symbole und Indizes gilt:
Ar ist bei jedem Auftreten gleich oder verschieden eine Aryl- oder Heteroarylgruppe mit 5 bis 60 aromatischen Ringatomen, die mit einem oder mehreren Resten R¹ substituiert sein kann, wobei zwei Reste R auch miteinander ein Ringsystem bilden können;
Z^{d} ist eine reaktive Gruppe B(R^{1a})₂;
R^{1a} ist bei jedem Auftreten gleich oder verschieden OH, eine geradkettige Alkyl- oder Alkoxygruppe mit 1 bis 20 C-Atomen, eine verzweigte oder cyclische Alkyl- oder Alkoxygruppe mit 3 bis 20 C-Atomen, wobei die lineare, verzweigte oder cyclische Alkyl- oder Alkoxygruppe jeweils mit einem oder mehreren Resten R² substituiert sein kann, oder ein aromatisches oder heteroaromatisches Ringsystem mit 5 bis 40 aromatischen Ringatomen, das jeweils durch einen oder mehrere Reste R² substituiert sein kann, dabei können zwei oder mehrere Reste R^{1a} miteinander ein Ringsystem bilden
wobei R² die für die Formel (I) genannte Bedeutung aufweist;
B) Umsetzung des Zwischenprodukts (ZA) mit einer zweiten reaktiven aromatischen oder heteroaromatischen Verbindung (B), die sich von der ersten reaktiven aromatischen oder heteroaromatischen Verbindung (A) unterscheidet, unter Erhalt einer unsymmetrisch substituierten stickstoffhaltigen heterocyclischen Verbindung
**dadurch gekennzeichnet, dass** die in Schritt B) umgesetzte Gruppe eine Gruppe der Formel OR ist und die in Schritt B) eingesetzte zweite reaktive aromatischen oder heteroaromatischen Verbindung (B) eine metallorganische Aryl- oder Heteroarylverbindung gemäß der Formel (B) ist
M-Ar Formel (B)
wobei für die verwendeten Symbole gilt:
Ar ist bei jedem Auftreten gleich oder verschieden eine Aryl- oder Heteroarylgruppe mit 5 bis 60 aromatischen Ringatomen, die mit einem oder mehreren Resten R¹ substituiert sein kann, wobei zwei Reste R¹ auch miteinander ein Ringsystem bilden können und R¹ die für die Formel (I) genannte Bedeutung aufweist;
M ist ein Metallatom, vorzugsweise Li, oder ein metallorganischer Rest, vorzugsweise MgBr oder MgCl,
und wobei die Umsetzung mit der metallorganischen Aryl- oder Heteroarylverbindung ohne den Zusatz von Übergangsmetall-Katalysatoren durchgeführt wird.

2. Verfahren gemäß Anspruch 1, **dadurch gekennzeichnet, dass** zwei der Gruppen Z^{a}, Z^{b}, Z^{c} gleich sind.

3. Verfahren gemäß Anspruch 1 oder 2, **dadurch gekennzeichnet, dass** mindestens eine der Gruppen Z^{a}, Z^{b}, Z^{c} eine Alkoxy-Gruppe oder eine Aryloxy-Gruppe mit bevorzugt 1 bis 20 C-Atomen, besonders bevorzugt 1 bis 10 C-Atomen, vorzugsweise eine Methoxy-, Ethoxy-, Propoxy- oder Aryloxy-Gruppe, besonders bevorzugt eine Methoxy-, Ethoxy- oder Phenoxy-Gruppe, speziell bevorzugt eine Methoxy- oder Phenoxy-Gruppe ist.

4. Verfahren nach Anspruch 3, **dadurch gekennzeichnet, dass** mindestens eine der Gruppen Z^{a}, Z^{b}, Z^{c} Cl, Br oder I, vorzugsweise Cl oder Br, besonders bevorzugt Cl ist.

5. Verfahren nach Anspruch 4, **dadurch gekennzeichnet, dass** mindestens eine der Gruppen Z^{a}, Z^{b}, Z^{c} Cl ist und mindestens eine der Gruppen Z^{a}, Z^{b}, Z^{c} eine Methoxy- oder Phenoxy-Gruppe ist, vorzugsweise zwei der Gruppen Z^{a}, Z^{b}, Z^{c} eine Methoxy- oder Phenoxy-Gruppe darstellen.

6. Verfahren nach einem der vorhergehenden Ansprüche, **dadurch gekennzeichnet, dass** ein Edukt gemäß der folgenden Formel (IV) eingesetzt wird 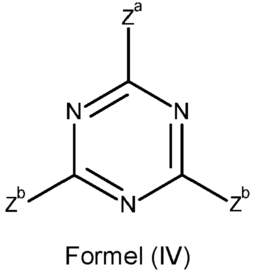 wobei die Symbole die in Anspruch 1 genannte Bedeutung aufweisen und die zwei Reste Z^{b} gleich sind.

7. Verfahren nach Anspruch 6, **dadurch gekennzeichnet, dass** Z^{a} ein Halogen ist, vorzugsweise Cl, Br oder I, vorzugsweise Cl oder Br, besonders bevorzugt Cl, und Z^{b} OR darstellt, wobei R die in Anspruch 1 genannte Bedeutung aufweist, wobei Z^{b} bevorzugt eine Alkoxy-Gruppe oder eine Aryloxy-Gruppe mit bevorzugt 1 bis 20 C-Atomen, besonders bevorzugt 1 bis 10 C-Atomen, insbesondere eine Methoxy-, Ethoxy-, Propoxy- oder Phenoxy-Gruppe, vorzugsweise eine Methoxy-, Ethoxy- oder Phenoxy-Gruppe, besonders bevorzugt eine Methoxy- oder Phenoxy-Gruppe ist.

8. Verfahren nach Anspruch 6, **dadurch gekennzeichnet, dass** Z^{a} OR darstellt, wobei R die in Anspruch 1 genannte Bedeutung aufweist, wobei Z^{a} bevorzugt eine Alkoxy-Gruppe oder eine Aryloxy-Gruppe mit bevorzugt 1 bis 20 C-Atomen, besonders bevorzugt 1 bis 10 C-Atomen, insbesondere eine Methoxy-, Ethoxy-, Propoxy- oder Phenoxy-Gruppe, vorzugsweise eine Methoxy-, Ethoxy- oder Phenoxy-Gruppe, besonders bevorzugt eine Methoxy- oder Phenoxy-Gruppe ist, und Z^{b} ein Halogen ist, vorzugsweise Cl, Br oder I, vorzugsweise Cl oder Br, besonders bevorzugt Cl.

9. Verfahren nach einem oder mehreren der vorhergehenden Ansprüche, **dadurch gekennzeichnet, dass** die Schritte A) und B) bei Temperaturen unterhalb von 140°C, vorzugsweise unterhalb von 120°C durchgeführt werden.

10. Verfahren nach einem oder mehreren der Ansprüche 1 bis 9, **dadurch gekennzeichnet, dass** ein organisch funktionales Material (OSM) hergestellt wird, welches zur Herstellung von Funktionsschichten elektronischer Vorrichtungen einsetzbar ist, welches vorzugsweise ausgewählt ist aus der Gruppe bestehend aus fluoreszierenden Emittern, phosphoreszierenden Emittern, Emittern, die TADF (thermally activated delayed fluorescence) zeigen, Hostmaterialien, Elektronentransportmaterialien, Excitonenblockiermaterialien, Elektroneninjektionsmaterialien, Lochleitermaterialien, Lochinjektionsmaterialien, n-Dotanden, p-Dotanden, Wide-Band-Gap-Materialien, Elektronenblockiermaterialien und/oder Lochblockiermaterialien.

11. Verfahren nach einem oder mehreren der Ansprüche 1 bis 10, **dadurch gekennzeichnet, dass** ein substituierter stickstoffhaltiger Heterocyclus hergestellt wird, die erste reaktive aromatische oder heteroaromatische Verbindung (A) oder die zweite reaktiven aromatischen oder heteroaromatischen Verbindung (B) ausgewählt ist aus der Gruppe bestehend aus der Gruppe der Fluorene, Indenofluorene, Spirobifluorene, Carbazole, Indenocarbazole, Indolocarbazole, Spirocarbazole, Pyrimidine, Triazine, Lactame, Triarylamine, Dibenzofurane, Dibenzothiene, Imidazole, Benzimidazole, Benzoxazole, Benzthiazole, 5-Aryl-Phenanthridin-6-one, 9,10-Dehydrophenanthrene, Fluoranthene.

## Claims

1. Process for the preparation of asymmetrically substituted nitrogen-containing heterocycles comprising the steps:
A) reaction of a starting material of the following formula (I) 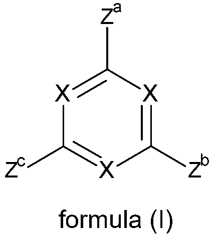
where the following applies to the symbols and indices used:
Z^{a}, Z^{b}, Z^{c} are halogen or OR;
X is on each occurrence, identically or differently, CR¹ or N, preferably N, with the proviso that at least two of the symbols X stand for N and especially preferably all symbols X stand for N;
R is on each occurrence, identically or differently, S(=O)₂R², a straight-chain alkyl group having 1 to 20 C atoms, a branched or cyclic alkyl group having 3 to 20 C atoms, where the linear, branched or cyclic alkyl group may in each case be substituted by one or more radicals R², or an aromatic or heteroaromatic ring system having 5 to 40 aromatic ring atoms, which may in each case be substituted by one or more radicals R²;
R¹ is on each occurrence, identically or differently, a straight-chain alkyl group having 1 to 20 C atoms, a branched or cyclic alkyl group having 3 to 20 C atoms, where the linear, branched or cyclic alkyl group may in each case be substituted by one or more radicals R², or an aromatic or heteroaromatic ring system having 5 to 40 aromatic ring atoms, which may in each case be substituted by one or more radicals R², two or more radicals R¹ may form a ring system with one another;
R² is on each occurrence, identically or differently, H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, a straight-chain alkyl, alkoxy or thioalkoxy group having 1 to 20 C atoms or an alkenyl or alkynyl group having 2 to 20 C atoms or a branched or cyclic alkyl, alkoxy or thioalkoxy group having 3 to 20 C atoms, where the alkyl, alkoxy, thioalkoxy, alkenyl or alkynyl group may in each case be substituted by one or more radicals R³, where one or more non-adjacent CH₂ groups may be replaced by R³C=CR³, C≡C, Si(R³)₂, C=O, NR³, O, S or CONR³, or an aromatic or heteroaromatic ring system having 5 to 40 aromatic ring atoms, which may in each case be substituted by one or more radicals R³, or an aryloxy or heteroaryloxy group having 5 to 40 aromatic ring atoms, which may be substituted by one or more radicals R³, or an aralkyl or heteroaralkyl group having 5 to 40 aromatic ring atoms, which may be substituted by one or more radicals R³, or a diarylamino group, diheteroarylamino group or arylheteroarylamino group having 10 to 40 aromatic ring atoms, which may be substituted by one or more radicals R³; two or more radicals R² may form a ring system with one another;
R³ is on each occurrence, identically or differently, H, D, F or an aliphatic, aromatic and/or heteroaromatic organic radical, in particular a hydrocarbon radical, having 1 to 20 C atoms, in which, in addition, one or more H atoms may be replaced by F, two or more substituents R³ may also form a mono- or polycyclic ring system with one another;
with the proviso that the group Z^{a} is not equal to the group Z^{b} or Z^{c} and at least one of the groups Z^{a}, Z^{b}, Z^{c} is a halogen; with a first reactive aromatic or heteroaromatic compound (A), giving an intermediate (ZA), where the first reactive aromatic or heteroaromatic compound (A) is an aryl or heteroaryl compound of the formula (A) 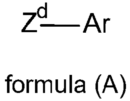
where the following applies to the symbols and indices used:
Ar is on each occurrence, identically or differently, an aryl or heteroaryl group having 5 to 60 aromatic ring atoms, which may be substituted by one or more radicals R¹, where two radicals R may also form a ring system with one another;
Z^{d} is a reactive group B(R^{1a})₂;
R^{1a} is on each occurrence, identically or differently, OH, a straight-chain alkyl or alkoxy group having 1 to 20 C atoms, a branched or cyclic alkyl or alkoxy group having 3 to 20 C atoms, where the linear, branched or cyclic alkyl or alkoxy group may in each case be substituted by one or more radicals R², or an aromatic or heteroaromatic ring system having 5 to 40 aromatic ring atoms, which may in each case be substituted by one or more radicals R², two or more radicals R^{1a} may form a ring system with one another where R² has the meaning given for the formula (I);
B) reaction of the intermediate (ZA) with a second reactive aromatic or heteroaromatic compound (B), which is different from the first reactive aromatic or heteroaromatic compound (A), giving an asymmetrically substituted nitrogen-containing heterocyclic compound,
**characterised in that** the group reacted in step B) is a group of the formula OR and the second reactive aromatic or heteroaromatic compound (B) employed in step B) is an organometallic aryl or heteroaryl compound of the formula (B)
M-Ar formula (B)
where the following applies to the symbols used:
Ar is on each occurrence, identically or differently, an aryl or heteroaryl group having 5 to 60 aromatic ring atoms, which may be substituted by one or more radicals R¹, where two radicals R¹ may also form a ring system with one another and R¹ has the meaning given for the formula (I);
M is a metal atom, preferably Li, or an organometallic radical, preferably MgBr or MgCl,
and where the reaction with the organometallic aryl or heteroaryl compound is carried out without the addition of transition-metal catalysts.

2. Process according to Claim 1, **characterised in that** two of the groups Z^{a}, Z^{b}, Z^{c} are identical.

3. Process according to Claim 1 or 2, **characterised in that** at least one of the groups Z^{a}, Z^{b}, Z^{c} is an alkoxy group or an aryloxy group, preferably having 1 to 20 C atoms, particularly preferably 1 to 10 C atoms, preferably a methoxy, ethoxy, propoxy or aryloxy group, particularly preferably a methoxy, ethoxy or phenoxy group, especially preferably a methoxy or phenoxy group.

4. Process according to Claim 3, **characterised in that** at least one of the groups Z^{a}, Z^{b}, Z^{c} is Cl, Br or I, preferably Cl or Br, particularly preferably Cl.

5. Process according to Claim 4, **characterised in that** at least one of the groups Z^{a}, Z^{b}, Z^{c} is Cl and at least one of the groups Z^{a}, Z^{b}, Z^{c} is a methoxy or phenoxy group, preferably two of the groups Z^{a}, Z^{b}, Z^{c} represent a methoxy or phenoxy group.

6. Process according to one of the preceding claims, **characterised in that**
a starting material of the following formula (IV) is employed 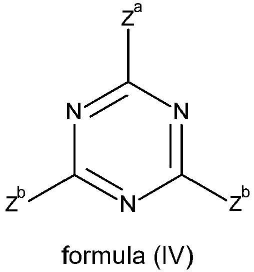
where the symbols have the meaning given in Claim 1 and the two radicals Z^{b} are identical.

7. Process according to Claim 6, **characterised in that** Z² is a halogen, preferably Cl, Br or I, preferably Cl or Br, particularly preferably Cl, and Z^{b} represents OR, where R has the meaning given in Claim 1, where Z^{b} is preferably an alkoxy group or an aryloxy group, preferably having 1 to 20 C atoms, particularly preferably 1 to 10 C atoms, in particular a methoxy, ethoxy, propoxy or phenoxy group, preferably a methoxy, ethoxy or phenoxy group, particularly preferably a methoxy or phenoxy group.

8. Process according to Claim 6, **characterised in that** Z^{a} represents OR, where R has the meaning given in Claim 1, where Z^{a} is preferably an alkoxy group or an aryloxy group, preferably having 1 to 20 C atoms, particularly preferably 1 to 10 C atoms, in particular a methoxy, ethoxy, propoxy or phenoxy group, preferably a methoxy, ethoxy or phenoxy group, particularly preferably a methoxy or phenoxy group, and Z^{b} is a halogen, preferably Cl, Br or I, preferably Cl or Br, particularly preferably Cl.

9. Process according to one or more of the preceding claims, **characterised in that** steps A) and B) are carried out at temperatures below 140°C, preferably below 120°C.

10. Process according to one or more of Claims 1 to 9, **characterised in that** an organofunctional material (OSM) is prepared which can be employed for the preparation of functional layers of electronic compounds, which is preferably selected from the group consisting of fluorescent emitters, phosphorescent emitters, emitters which exhibit TADF (thermally activated delayed fluorescence), host materials, electron-transport materials, exciton-blocking materials, electron-injection materials, hole-conductor materials, hole-injection materials, n-dopants, p-dopants, wide-band-gap materials, electron-blocking materials and/or hole-blocking materials.

11. Process according to one or more of Claims 1 to 10, **characterised in that** a substituted nitrogen-containing heterocycle is prepared, the first reactive aromatic or heteroaromatic compound (A) or the second reactive aromatic or heteroaromatic compound (B) is selected from the group consisting of the group of the fluorenes, indenofluorenes, spiro-bifluorenes, carbazoles, indenocarbazoles, indolocarbazoles, spiro-carbazoles, pyrimidines, triazines, lactams, triarylamines, dibenzo-furans, dibenzothienes, imidazoles, benzimidazoles, benzoxazoles, benzothiazoles, 5-arylphenanthridin-6-ones, 9,10-dehydrophen-anthrenes, fluoranthenes.

## Revendications

1. Procédé de préparation d'hétérocycles azotés à substitution asymétrique, comprenant les étapes :
A) de réaction d'un matériau de départ de formule (I) suivante 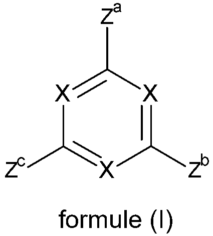
dans laquelle ce qui suit s'applique aux symboles et indices utilisés :
Z^{a}, Z^{b}, Z^{c} sont halogène ou OR ;
X est à chaque occurrence, de manière identique ou différente, CR¹ ou N, préférablement N, à condition qu'au moins deux parmi les symboles X représentent N et particulièrement préférablement tous les symboles X représentent N ;
R est à chaque occurrence, de manière identique ou différente, S(=O)₂R², un groupement alkyle à chaîne linéaire ayant de 1 à 20 atomes de C, un groupement alkyle ramifié ou cyclique ayant de 3 à 20 atomes de C, où le groupement alkyle linéaire, ramifié ou cyclique peut dans chaque cas être substitué par un ou plusieurs radicaux R², ou un noyau aromatique ou hétéroaromatique ayant de 5 à 40 atomes de cycle aromatique, pouvant dans chaque cas être substitué par un ou plusieurs radicaux R² ;
R¹ est à chaque occurrence, de manière identique ou différente, un groupement alkyle à chaîne linéaire ayant de 1 à 20 atomes de C, un groupement alkyle ramifié ou cyclique ayant de 3 à 20 atomes de C, où le groupement alkyle linéaire, ramifié ou cyclique peut dans chaque cas être substitué par un ou plusieurs radicaux R², ou un noyau aromatique ou hétéroaromatique ayant de 5 à 40 atomes de cycle aromatique, pouvant dans chaque cas être substitué par un ou plusieurs radicaux R², deux, ou plus, radicaux R¹ peuvent former un noyau les uns avec les autres ;
R² est à chaque occurrence, de manière identique ou différente, H, D, F, Cl, Br, I, N(R³)₂, CN, NO₂, Si(R³)₃, B(OR³)₂, C(=O)R³, P(=O)(R³)₂, S(=O)R³, S(=O)₂R³, OSO₂R³, un groupement alkyle, alcoxy ou thioalcoxy à chaîne linéaire ayant de 1 à 20 atomes de C ou un groupement alcényle ou alcynyle ayant de 2 à 20 atomes de C ou un groupement alkyle, alcoxy ou thioalcoxy ramifié ou cyclique ayant de 3 à 20 atomes de C, où le groupement alkyle, alcoxy, thioalcoxy, alcényle ou alcynyle peut être dans chaque cas substitué par un ou plusieurs radicaux R³, où un ou plusieurs groupements CH₂ non adjacents peuvent être remplacés par R³C=CR³, C≡C, Si(R³)₂, C=O, NR³, O, S ou CONR³, ou un noyau aromatique ou hétéroaromatique ayant de 5 à 40 atomes de cycle aromatique, pouvant dans chaque cas être substitué par un ou plusieurs radicaux R³, ou un groupement aryloxy ou hétéroaryloxy ayant de 5 à 40 atomes de cycle aromatique, qui peut être substitué par un ou plusieurs radicaux R³, ou un groupement aralkyle ou hétéroaral-kyle ayant de 5 à 40 atomes de cycle aromatique, qui peut être substitué par un ou plusieurs radicaux R³, ou un groupement diarylamino, un groupement dihétéroarylamino ou un groupement arylhétéroarylamino ayant de 10 à 40 atomes de cycle aromatique, qui peut être substitué par un ou plusieurs radicaux R³ ; deux, ou plus, radicaux R² peuvent former un noyau les uns avec les autres ;
R³ est à chaque occurrence, de manière identique ou différente, H, D, F ou un radical organique aliphatique, aromatique et/ou hétéroaromatique, en particulier un radical hydrocarboné, ayant de 1 à 20 atomes de C, où, de plus, un ou plusieurs atomes de H peuvent être remplacés par F, deux, ou plus, substituants R³ peuvent également former un noyau mono- ou polycyclique les uns avec les autres ;
à condition que le groupement Z^{a} ne soit pas équivalent au groupement Z^{b} ou Z^{c} et qu'au moins l'un parmi les groupements Z^{a}, Z^{b}, Z^{c} soit un halogène ;
avec un premier composé aromatique ou hétéroaromatique réactif (A), pour conduire à un intermédiaire (ZA), le premier composé aromatique ou hétéroaromatique réactif (A) étant un composé aryle ou hétéroaryle de formule (A) 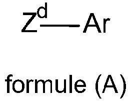 dans laquelle ce qui suit s'applique aux symboles et indices utilisés :
Ar est à chaque occurrence, de manière identique ou différente, un groupement aryle ou hétéroaryle ayant de 5 à 60 atomes de cycle aromatique, qui peut être substitué par un ou plusieurs radicaux R¹, où deux radicaux R peuvent également former un noyau l'un avec l'autre ;
Z^{d} est un groupement réactif B(R^{1a})₂ ;
R^{1a} est à chaque occurrence, de manière identique ou différente, OH, un groupement alkyle ou alcoxy à chaîne linéaire ayant de 1 à 20 atomes de C, un groupement alkyle ou alcoxy ramifié ou cyclique ayant de 3 à 20 atomes de C, où le groupement alkyle ou alcoxy linéaire, ramifié ou cyclique peut être dans chaque cas substitué par un ou plusieurs radicaux R², ou un noyau aromatique ou hétéroaromatique ayant de 5 à 40 atomes de cycle aromatique, pouvant dans chaque cas être substitué par un ou plusieurs radicaux R², deux, ou plus, radicaux R^{1a} peuvent former un noyau les uns avec les autres
où R² revêt la signification donnée pour la formule (I) ;
B) de réaction de l'intermédiaire (ZA) avec un deuxième composé aromatique ou hétéroaromatique réactif (B), qui est différent du premier composé aromatique ou hétéroaromatique réactif (A), pour conduire à un composé hétérocyclique azoté à substitution asymétrique,
**caractérisé en ce que** le groupement réagi dans l'étape B) est un groupement de formule ou et le deuxième composé aromatique ou hétéroaromatique réactif (B) employé dans l'étape B) est un composé aryle ou hétéroaryle organométallique de formule (B)
M-Ar formule (B)
dans laquelle ce qui suit s'applique aux symboles utilisés :
Ar est à chaque occurrence, de manière identique ou différente, un groupement aryle ou hétéroaryle ayant de 5 à 60 atomes de cycle aromatique, qui peut être substitué par un ou plusieurs radicaux R¹, où deux radicaux R¹ peuvent également former un noyau l'un avec l'autre et R¹ revêt la signification donnée pour la formule (I) ;
M est un atome de métal, préférablement Li, ou un radical organométallique, préférablement MgBr ou MgCl,
et où la réaction avec le composé aryle ou hétéroaryle organométallique est effectuée sans l'addition de catalyseurs à base de métaux de transition.

2. Procédé selon la revendication 1, **caractérisé en ce que** deux parmi les groupements Z^{a}, Z^{b}, Z^{c} sont identiques.

3. Procédé selon la revendication 1 ou 2, **caractérisé en ce qu'**au moins l'un parmi les groupements Z^{a}, Z^{b}, Z^{c} est un groupement alcoxy ou un groupement aryloxy, préférablement ayant de 1 à 20 atomes de C, particulièrement préférablement de 1 à 10 atomes de C, préférablement un groupement méthoxy, éthoxy, propoxy ou aryloxy, particulièrement préférablement un groupement méthoxy, éthoxy ou phénoxy, particulièrement préférablement un groupement méthoxy ou phénoxy.

4. Procédé selon la revendication 3, **caractérisé en ce qu'**au moins l'un parmi les groupements Z^{a}, Z^{b}, Z^{c} est Cl, Br ou I, préférablement Cl ou Br, particulièrement préférablement Cl.

5. Procédé selon la revendication 4, **caractérisé en ce qu'**au moins l'un parmi les groupements Z^{a}, Z^{b}, Z^{c} est Cl et qu'au moins l'un parmi les groupements Z^{a}, Z^{b}, Z^{c} est un groupement méthoxy ou phénoxy, préférablement deux parmi les groupements Z^{a}, Z^{b}, Z^{c} représentent un groupement méthoxy ou phénoxy.

6. Procédé selon l'une parmi les revendications précédentes, **caractérisé en ce qu'**un matériau de départ de formule (IV) suivante est employé 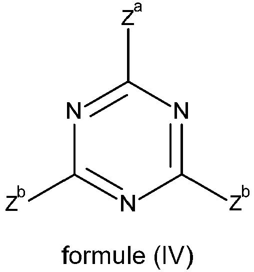 dans laquelle les symboles revêtent la signification donnée selon la revendication 1 et les deux radicaux Z^{b} sont identiques.

7. Procédé selon la revendication 6, **caractérisé en ce que** Z^{a} est un halogène, préférablement Cl, Br ou I, préférablement Cl ou Br, particulièrement préférablement Cl, et Z^{b} représente OR, où R revêt la signification donnée selon la revendication 1, où Z^{b} est préférablement un groupement alcoxy ou un groupement aryloxy, préférablement ayant de 1 à 20 atomes de C, particulièrement préférablement de 1 à 10 atomes de C, en particulier un groupement méthoxy, éthoxy, propoxy ou phénoxy, préférablement un groupement méthoxy, éthoxy ou phénoxy, particulièrement préférablement un groupement méthoxy ou phénoxy.

8. Procédé selon la revendication 6, **caractérisé en ce que** Z^{a} représente OR, où R revêt la signification donnée selon la revendication 1, où Z^{a} est préférablement un groupement alcoxy ou un groupement aryloxy, préférablement ayant de 1 à 20 atomes de C, particulièrement préférablement de 1 à 10 atomes de C, en particulier un groupement méthoxy, éthoxy, propoxy ou phénoxy, préférablement un groupement méthoxy, éthoxy ou phénoxy, particulièrement préférablement un groupement méthoxy ou phénoxy, et Z^{b} est un halogène, préférablement Cl, Br ou I, préférablement Cl ou Br, particulièrement préférablement Cl.

9. Procédé selon l'une ou plusieurs parmi les revendications précédentes, **caractérisé en ce que** les étapes A) et B) sont effectuées à des températures inférieures à 140°C, préférablement inférieures à 120°C.

10. Procédé selon l'une ou plusieurs parmi les revendications 1 à 9, **caractérisé en ce qu'**un matériau organique fonctionnel (OSM) est préparé, lequel peut être employé pour la préparation de couches fonctionnelles de composés électroniques, qui est préférablement choisi dans le groupe constitué par les émetteurs fluorescents, les émetteurs phosphorescents, les émetteurs présentant une TADF (fluorescence retardée activée thermiquement), les matériaux hôtes, les matériaux de transport d'électrons, les matériaux de blocage d'excitons, les matériaux d'injection d'électrons, les matériaux conducteurs de trous, les matériaux d'injection de trous, les dopants de type N, les dopants de type P, les matériaux à large bande interdite, les matériaux de blocage d'électrons et/ou les matériaux de blocage de trous.

11. Procédé selon l'une ou plusieurs parmi les revendications 1 à 10, **caractérisé en ce qu'**un hétérocycle azoté substitué est préparé, le premier composé aromatique ou hétéroaromatique réactif (A) ou le deuxième composé aromatique ou hétéroaromatique réactif (B) est choisi dans le groupe constitué par les fluorènes, les indénofluorènes, les spirobifluorènes, les carbazoles, les indénocarbazoles, les indolo-carbazoles, les spirocarbazoles, les pyrimidines, les triazines, les lactames, les triarylamines, les dibenzofuranes, les dibenzothiènes, les imidazoles, les benzimidazoles, les benzoxazoles, les benzothiazoles, les 5-arylphénanthridin-6-ones, les 9,10-déshydrophénanthrènes, les fluoranthènes.
